# EUROPEAN PATENT APPLICATION

(11) **EP 4 825 869 A1**
(43) Date of publication of application: **21.10.2026**
(21) Application number: 24902953.9
(22) Date of filing: 13.12.2024
(51) Int. Cl.: C07K 14/725, A61K 38/16, A61P 35/00, A61P 37/02, A61P 37/06

(54) **CHEMOKINE RECEPTOR 8 ANTAGONISTIC POLYPEPTIDE COMPOUND, COMPOSITION, KIT, AND USE**

(30) Priority: 15.12.2023 CN 202311730072; 29.07.2024 CN 202411029728
(71) Applicant: Jiangsu Kanion Pharmaceutical Co., Ltd., Lianyungang, Jiangsu 222047 (CN)
(72) Inventor: XIAO, Wei, Lianyungang, Jiangsu 222047 (CN); ZHOU, Jianming, Lianyungang, Jiangsu 222047 (CN); SONG, Ying, Lianyungang, Jiangsu 222047 (CN); HAO, Shaohua, Lianyungang, Jiangsu 222047 (CN); HE, Runze, Lianyungang, Jiangsu 222047 (CN); WANG, Zhenzhong, Lianyungang, Jiangsu 222047 (CN); ZHANG, Chenfeng, Lianyungang, Jiangsu 222047 (CN)
(74) Representative: Geling, Andrea
(86) International application number: PCT/CN2024/139107
(87) International publication number: WO 2025/124536

(57) **Abstract**

Provided in the present application are a CCR8 antagonistic polypeptide compound, a pharmaceutical composition thereof, a kit thereof, the use of the compound, and the use of the pharmaceutical composition. The CCR8 antagonistic polypeptide compound can be used for preventing or treating cancers or tumors, inflammatory diseases, viral infections, IgG4-related diseases, cutaneous immune diseases, or related symptoms thereof.

## Description

### TECHNICAL FIELD

The present application pertains to the field of medicine. Specifically, the present application relates to a chemokine receptor 8 (CCR8) antagonistic polypeptide compound, a pharmaceutical composition and a kit comprising the same, and use thereof, especially for treating and/or preventing cancers or tumors, inflammatory diseases, viral infections, IgG4-related diseases, immunologic skin diseases, or related symptoms thereof (e.g., immunologic skin diseases related to CCL1-CCR8 signaling pathway, or related symptoms thereof).

### BACKGROUND

CCR8 (Chemokine receptor 8) is a seven-transmembrane G protein-coupled receptor belonging to the C-C subfamily of chemokine receptors. Although the gene encoding CCL1 is located within this chemokine gene cluster, it is the only CC chemokine within the cluster that specifically binds to CCR8, in other words, it possesses a single ligand/receptor relationship. During *in vitro* studies, the binding and cross-linking of IgE on mast cells lead to significant upregulation of the inflammatory chemokine CCL1, and its specific receptor, CCR8, is expressed on a small subset of circulating T cells and is abundantly expressed on interstitial dendritic cells, *in vitro*-generated Langerhans cells, and their monocyte precursors. Although dendritic cells maintain their CCR8 status during maturation, transient activation of circulating T cells recruits CCR8 from cytoplasmic reserves to the cell surface. Immunologic skin diseases, also known as cutaneous immune diseases, are a class of skin diseases caused by immune imbalance resulting in skin damage, including, for example, inflammatory skin diseases (a group of diseases caused by skin tissue destruction due to immune system dysregulation, including eczema, atopic dermatitis, psoriasis, chronic urticaria, etc.), and autoimmune skin diseases (various skin diseases caused by autoimmune disorders where the immune system no longer protects the body from bacteria and viruses but instead attacks its own tissues, such as connective tissue diseases (lupus erythematosus, scleroderma, dermatomyositis, etc.), cutaneous vasculitis (allergic vasculitis, atrophie blanche, Henoch-Schönlein purpura, urticarial vasculitis), and autoimmune bullous diseases (pemphigus, bullous pemphigoid, dermatitis herpetiformis, epidermolysis bullosa, etc.)).

Atopic dermatitis (AD) is a chronic relapsing skin disease with a steadily increasing prevalence in children, reaching 10%-20%. Skin-infiltrating T cells, dendritic cells (DCs), and mast cells play key roles in its pathogenesis. Compared with psoriasis, cutaneous lupus erythematosus or normal skin, the expression of the CC chemokine CCL1 (I-309) is significantly and selectively upregulated in atopic dermatitis. Serum levels of CCL1 in patients with atopic dermatitis are significantly higher than those in healthy individuals. Studies have shown that CCL1-CCR8 links adaptive and innate immune functions, which play roles in the initiation and amplification of atopic dermatitis.

Atopic dermatitis has a complex pathophysiology underlying multiple clinical phenotypes. AD is still difficult to treat due to the limited response to existing therapies. Starting from the mechanism that CCL1-CCR8 axis promotes the progression of atopic dermatitis, the present application develops polypeptides that specifically bind to CCR8 and block the CCL1-CCR8 signaling, thereby achieving the effect of treating/preventing atopic dermatitis. The polypeptides have high safety for humans or animals, making them more suitable for atopic dermatitis, a chronic disease requiring long-term medication.

### SUMMARY OF THE INVENTION

The present application provides a polypeptide compound capable of specifically binding to CCR8 and blocking the CCL1 (ligand)-CCR8 (receptor) signaling pathway, which thereby can be used as an active ingredient in a therapeutic and/or prophylactic agent for cancers or tumors, inflammatory diseases, viral infections, IgG4-related diseases, and cutaneous immune diseases.

In one aspect, the present application provides a CCR8 antagonistic polypeptide compound, comprising an amino acid sequence selected from:
(1) H₂N-AIB-Arg-Pro-Asp-Lys-Ala-Ala-Leu-Gly-Tyr-Gln-Lys-Arg-Pro-Leu-Pro-Gln-Xaa₁-Xaa₂-Xaa₃-Xaa₄-Xaa₅-Xaa₆-CONH₂ (SEQ ID NO: 1),
   wherein Xaa₁ is Val, Ala, Leu, or Ile, Xaa₂ is Leu, or Val, Xaa₃ is Leu, Val, or Ile, Xaa₄ is Thr, Ser, Ala, or Ile, Xaa₅ is Ser, Thr, or Ala, and Xaa₆ is Ile, Val, Leu, AIB, Cys, Nle, Ala, Met, or Abu (e.g., Xaa₆ is Val, Leu, AIB, Cys, Nle, Ala, Met, or Abu; or Xaa₆ is Ile, Val, AIB, Cys, Nle, Ala, Met, or Abu);
(2) H₂N-AIB-Arg-Pro-Asp-Lys-Ala-Ala-Leu-Gly-Tyr-Gln-Lys-Arg-Pro-Ile-Pro-Asn-Ile-Xaa₇-Xaa₈-Xaa₉-Xaa₁₀-Xaa₁₁-CONH₂ (SEQ ID NO: 2),
   wherein Xaa₇ is Leu, or Val, Xaa₈ is Leu, Val, or Ile, Xaa₉ is Thr, Ala, or Ser, Xaa₁₀ is Thr, Ala, or Ser, and Xaa₁₁ is Leu, Val, AIB, Cys, Nle, Ala, Met, or Abu;
(3) H₂N-AIB-Arg-Pro-Asp-Lys-Ala-Ala-Leu-Gly-Tyr-Asn-Orn-Arg-Pro-Ile-Pro-Asn-Ile-Xaa₁₂-Xaa₁₃-Xaa₁₄-Xaa₁₅-Xaa₁₆-CONH₂ (SEQ ID NO: 3),
   wherein Xaa₁₂ is Leu, or Val, Xaa₁₃ is Leu, Val, or Ile, Xaa₁₄ is Thr, Ala, or Ser, Xaa₁₅ is Thr, Ala, or Ser, and Xaa₁₆ is Leu, Val, AIB, Cys, Nle, Ala, Met, or Abu;
(4) H₂N-AIB-Arg-Pro-Xaa₁₇-Xaa₁₈-Xaa₁₉-Ala-Leu-Gly-Tyr-Asn-Orn-Arg-Pro-Leu-Pro-Gln-Val-Leu-Leu-Xaa₂₀-Xaa₂₁-Abu-CONH₂ (SEQ ID NO: 4),
   wherein Xaa₁₇-Xaa₁₈-Xaa₁₉ is Asp-Lys-Ala, or Glu-Orn-Ser, Xaa₂₀ is Thr, Ala, or Ser, Xaa₂₁ is Thr, or Ser; or
(5) H₂N-AIB-Arg-Pro-Glu-Orn-Ser-Ala-Leu-Gly-Tyr-Asn-Orn-Arg-Pro-Ile-Pro-Asn-Ile-Xaa₂₂-Xaa₂₃-Xaa₂₄-Xaa₂₅-Xaa₂₆-CONH₂ (SEQ ID NO: 5),
   wherein Xaa₂₂ is Leu, or Val, Xaa₂₃ is Leu, Val, or Ile, Xaa₂₄ is Thr, Ala, or Ser, Xaa₂₅ is Thr, Ala, or Ser, and Xaa₂₆ is Leu, Val, AIB, Cys, Nle, Ala, Met, or Abu;
   or an amino acid sequence having at least 80%, at least 85%, at least 90%, at least 91%, at least 92%, at least 93%, at least 94%, at least 95%, at least 96%, at least 97%, at least 98%, or at least 99% identity thereto.

In one aspect, the present application provides a polynucleotide, encoding the CCR8 antagonistic polypeptide compound.

In one aspect, the present application provides a pharmaceutical composition, comprising the polypeptide compound and a pharmaceutically acceptable excipient.

In one aspect, the present application provides a kit, comprising the polypeptide compound or the pharmaceutical composition thereof.

In one aspect, the present application provides use of the polypeptide compound, the pharmaceutical composition, or the kit in the preparation of a medicament for preventing or treating cancers or tumors, inflammatory diseases, viral infections, IgG4-related diseases, cutaneous immune diseases, or related symptoms thereof. Alternatively, the present application provides use of the polypeptide compound, the pharmaceutical composition, or the kit in the preparation of an agent for blocking the CCL1-CCR8 signaling pathway.

### DETAILED DESCRIPTION OF THE DRAWINGS

Fig. 1 is a mass spectrum of the purified TCR10 sample, wherein the HPLC purity of the polypeptide compound can reach 95.62%.
Fig. 2 is a graph of CCR8 expression on the surface of interstitial dendritic cells detected by flow cytometry in Experimental Example 2.
Fig. 3 is a graph of the migration ability of iDCs in the presence or absence of TCR10 detected by the Transwell chemotaxis assay in Experimental Example 2.
Fig. 4 is a graph showing the statistical results of migrated cell numbers in Experimental Example 2 (the results of the blank control group, positive control group, test compound group, and antibody control group are shown from left to right in the figure).
Fig. 5 is a graph showing the changes of mouse weight over time in the drug efficacy screening test of Experimental Example 3.
Fig. 6 is a graph showing the degree of mouse ear swelling in the drug efficacy screening test of Experimental Example 3.
Fig. 7 is a graph showing dorsal skin lesion scores of mice in the drug efficacy screening test of Experimental Example 3.
Fig. 8 shows the liver, kidney, and spleen indices of mice in the drug efficacy screening test of Experimental Example 3 (from left to right).
Fig. 9 shows the pathological section analysis of dorsal skin of mice in the drug efficacy screening test of Experimental Example 3.
Fig. 10 shows the pathological section analysis of mice ear in the drug efficacy screening test of Experimental Example 3.
Fig. 11 shows the skin condition of mice in the dose-effect test of Experimental Example 4: (A) control group; (B) model group; (C) 0.1% mometasone furoate group; (D) 20 mg crisaborole group; (E) TCR09 (1 mg) group; (F) TCR10 (1 mg) group; (G) TCR09 (3 mg) group; (H) TCR10 (3 mg) group.
Fig. 12 shows the changes of mouse weight over induction time in the gel formulation treatment test of Experimental Example 5.
Fig. 13 shows the change trend (%) of mouse weight in the gel formulation treatment test of Experimental Example 5.
Fig. 14 shows the skin lesion scores of mice over induction time in the gel formulation treatment test of Experimental Example 5.
Fig. 15 shows the skin lesion scores of mice at the time of the last administration (Day 14) in the gel formulation treatment test of Experimental Example 5 (**: p<0.01; ***: p<0.001).
Fig. 16 shows the degree of mouse ear swelling over induction time in the gel formulation treatment test of Experimental Example 5.
Fig. 17 shows the degree of mouse ear swelling at the time of the last administration (Day 14) in the gel formulation treatment test of Experimental Example 5 (***: p<0.001).
Fig. 18 shows the content of cytokine IL-4 per mg of mouse skin (pg/mg skin) in the gel formulation treatment test of Experimental Example 5 (*: p<0.05; **: p<0.01; ***: p<0.001).
Fig. 19 shows the HE scores of mice in the gel formulation treatment test of Experimental Example 5 (*: p<0.05; ***: p<0.001).
Fig. 20 shows the epidermal thickness of mice in the gel formulation treatment test of Experimental Example 5 (*: p<0.05; ***: p<0.001).
Fig. 21 shows the mast cell counts of mice in the gel formulation treatment test of Experimental Example 5 (*: p<0.05; ***: p<0.001).
Fig. 22 shows the pathological sections of mouse skin in the normal control group, model group, and gel matrix group in the gel formulation treatment test of Experimental Example 5 (Left: HE staining; Right: toluidine blue staining).
Fig. 23 shows the pathological sections of mouse skin in the mometasone furoate group, crisaborole group, and 2% TCR10 gel group in the gel formulation treatment test of Experimental Example 5 (Left: HE staining; Right: toluidine blue staining).
Fig. 24 shows the pathological sections of mouse skin in the 5% TCR10 gel group in the gel formulation treatment test of Experimental Example 5 (Left: HE staining; Right: toluidine blue staining).
Fig 25 shows the skin condition of mice in the gel formulation treatment test of Experimental Example 5, from left to right: normal control group, model group, gel matrix control group, mometasone furoate gel group, crisaborole ointment group, 2% TCR10 gel group, and 5% TCR10 gel group.

### EMBODIMENTS OF THE INVENTION

Next, the present application will be described in more details to facilitate the understanding of the technical solutions of the present application by those skilled in the art. However, it should be noted that the scope of protection of the present application is not limited thereto. Unless defined otherwise, all technical and scientific terms used herein have the same meanings as commonly understood by those skilled in the art.

In the present application, unless otherwise specified, the term "polypeptide compound" refers to a compound formed by multiple amino acids linked by peptide bonds. The polypeptide of the present disclosure may comprise natural amino acids, synthetic amino acids (e.g., 2-aminobutyric acid (Abu), 2-aminoisobutyric acid (AIB), norleucine (Nle)) or amino acid analogs, and chemical modifications (e.g., phospholipid modification).

Unless otherwise specified, the term "treatment" herein refers to curing, alleviating, relieving, slowing, palliating, or improving a disease or disease-related symptoms in a statistically significant manner, or preventing, delaying, stopping, suspending, or terminating the onset or further progression of a disease or related symptoms.

Unless otherwise specified, all numbers expressing quantities of ingredients, measurement values, or reaction conditions herein should be understood as being modified in all instances by the term "about" to indicate possible measurement errors. For example, when used in connection with a percentage, the term "about" can mean varying within a range of ±1%, for example, ±0.5% of the object value it defines.

Unless otherwise specified, singular terms herein encompass plural referents, and *vice versa*. Similarly, unless otherwise explicitly indicated in the context, the term "or" means to include "and", and *vice versa*.

Unless otherwise specified, the terms "comprise", "comprises" and "comprising" or their equivalents (e.g., "contain", "containing", "include", "including") herein are open-ended expressions, and should be understood as "including but not limited to", meaning that in addition to the listed elements, components and steps, other unspecified elements, components and steps may also be covered.

The terms "X" and "Xaa" herein are equivalent to each other and refer to an Unspecified Amino Acid, and the scope covered thereby is specified by the definitions in the relevant expressions. To distinguish multiple "Xs" in a single amino acid sequence, the multiple Xs that appear successively are numbered respectively (i.e., written as Xn) and the scope covered thereby is defined respectively.

The term "pharmaceutically acceptable" refers to those compounds, materials, compositions, and/or dosage forms that are, within the scope of sound medical judgment, suitable for use in contact with the tissues of human and animals without excessive toxicity, irritation, allergic response, or other problem or complication, commensurate with a reasonable benefit/risk ratio.

The term "subject" covers any vertebrate animal, for example, mammals and non-mammals, such as human, non-human primate (e.g., chimpanzee, *Macaca mulatta*, *Macaca fascicularis*, etc.), sheep, dog, cat, horse, cattle, chicken, pig, mouse, etc., preferably a human.

### CCR8 Antagonistic Polypeptide Compound

In the first aspect of the present disclosure, the present application provides a CCR8 antagonistic polypeptide compound, comprising an amino acid sequence selected from:
(1) H₂N-AIB-Arg-Pro-Asp-Lys-Ala-Ala-Leu-Gly-Tyr-Gln-Lys-Arg-Pro-Leu-Pro-Gln-Xaa₁-Xaa₂-Xaa₃-Xaa₄-Xaa₅-Xaa₆-CONH₂ (SEQ ID NO: 1),
   wherein Xaa₁ is Val, Ala, Leu, or Ile, Xaa₂ is Leu, or Val, Xaa₃ is Leu, Val, or Ile, Xaa₄ is Thr, Ser, Ala, or Ile, Xaa₅ is Ser, Thr, or Ala, and Xaa₆ is Ile, Val, Leu, AIB, Cys, Nle, Ala, Met, or Abu;
(2) H₂N-AIB-Arg-Pro-Asp-Lys-Ala-Ala-Leu-Gly-Tyr-Gln-Lys-Arg-Pro-Ile-Pro-Asn-Ile-Xaa₇-Xaa₈-Xaa₉-Xaa₁₀-Xaa₁₁-CONH₂ (SEQ ID NO: 2),
   wherein Xaa₇ is Leu, or Val, Xaa₈ is Leu, Val, or Ile, Xaa₉ is Thr, Ala, or Ser, Xaa₁₀ is Thr, Ala, or Ser, and Xaa₁₁ is Leu, Val, AIB, Cys, Nle, Ala, Met, or Abu;
(3) H₂N-AIB-Arg-Pro-Asp-Lys-Ala-Ala-Leu-Gly-Tyr-Asn-Orn-Arg-Pro-Ile-Pro-Asn-Ile-Xaa₁₂-Xaa₁₃-Xaa₁₄-Xaa₁₅-Xaa₁₆-CONH₂ (SEQ ID NO: 3),
   wherein Xaa₁₂ is Leu, or Val, Xaa₁₃ is Leu, Val, or Ile, Xaa₁₄ is Thr, Ala, or Ser, Xaa₁₅ is Thr, Ala, or Ser, and Xaa₁₆ is Leu, Val, AIB, Cys, Nle, Ala, Met, or Abu;
(4) H₂N-AIB-Arg-Pro-Xaa₁₇-Xaa₁₈-Xaa₁₉-Ala-Leu-Gly-Tyr-Asn-Orn-Arg-Pro-Leu-Pro-Gln-Val-Leu-Leu-Xaa₂₀-Xaa₂₁-Abu-CONH₂ (SEQ ID NO: 4),
   wherein Xaa₁₇-Xaa₁₈-Xaa₁₉ is Asp-Lys-Ala, or Glu-Orn-Ser, Xaa₂₀ is Thr, Ala, or Ser, and Xaa₂₁ is Thr, or Ser; or
(5) H₂N-AIB-Arg-Pro-Glu-Orn-Ser-Ala-Leu-Gly-Tyr-Asn-Orn-Arg-Pro-Ile-Pro-Asn-Ile-Xaa₂₂-Xaa₂₃-Xaa₂₄-Xaa₂₅-Xaa₂₆-CONH₂ (SEQ ID NO: 5),
   wherein Xaa₂₂ is Leu, or Val, Xaa₂₃ is Leu, Val, or Ile, Xaa₂₄ is Thr, Ala, or Ser, Xaa₂₅ is Thr, Ala, or Ser, and Xaa₂₆ is Leu, Val, AIB, Cys, Nle, Ala, Met, or Abu;
   or an amino acid sequence having at least 80%, at least 85%, at least 90%, at least 91%, at least 92%, at least 93%, at least 94%, at least 95%, at least 96%, at least 97%, at least 98%, or at least 99% identity thereto.

In some embodiments, Xaa₁-Xaa₂-Xaa₃ is Val-Leu-Leu. Further, Xaa₄ is Thr, Ser, or Ala. Further, Xaa₅ is Ser, Thr, or Ala. Furthermore, Xaa₆ is Ile, Val, Leu, AIB, Cys, Nle, Ala, Met, or Abu.

In some preferred embodiments, Xaa₁-Xaa₂-Xaa₃ is Val-Leu-Leu, Xaa₄ is Thr, or Ser, Xaa₅ is Ser, and Xaa₆ is Ile, Val, AIB, Cys, Nle, Ala, Met, or Abu. In some alternative preferred embodiments, Xaa₁-Xaa₂-Xaa₃ is Val-Leu-Leu, Xaa₄ is Thr, or Ser, Xaa₅ is Ser, Thr, or Ala, and Xaa₆ is Ile.

In some embodiments, Xaa₁-Xaa₂-Xaa₃-Xaa₄ is Val-Leu-Leu-Thr, Xaa₅ is Ser, Thr, or Ala, and Xaa₆ is Val, Leu, AIB, Cys, Nle, Ala, Met, or Abu. In some embodiments, Xaa₁-Xaa₂-Xaa₃-Xaa₄ is Val-Leu-Leu-Ser, Xaa₅ is Ser, or Thr, and Xaa₆ is Ile, Val, AIB, Cys, Nle, Ala, Met, or Abu.

In some embodiments, the CCR8 antagonistic polypeptide compound comprises the following amino acid sequence:
H₂N-AIB-Arg-Pro-Asp-Lys-Ala-Ala-Leu-Gly-Tyr-Gln-Lys-Arg-Pro-Leu-Pro-Gln-Val-Leu-Leu-Xaa₂₇-Ser-Val-CONH₂ (SEQ ID NO: 6), wherein Xaa₂₇ is Thr, or Ser.

In some embodiments, the CCR8 antagonistic polypeptide compound comprises the following amino acid sequence:
H₂N-AIB-Arg-Pro-Asp-Lys-Ala-Ala-Leu-Gly-Tyr-Gln-Lys-Arg-Pro-Leu-Pro-Gln-Val-Leu-Leu-Ser-Ser-Xaa₂₈-CONH₂ (SEQ ID NO: 206), wherein Xaa₂₈ is Ile, or Val.

In some embodiments, the CCR8 antagonistic polypeptide compound comprises the amino acid sequence set forth in any one selected from SEQ ID NO: 7 to SEQ ID NO: 205. In some preferred embodiments, the amino acid sequence of the CCR8 antagonistic polypeptide compound is set forth in SEQ ID NO: 16 or SEQ ID NO: 15.

In some embodiments, Xaa₇-Xaa₈ is Leu-Leu. Further, Xaa₉ is Thr, or Ser, Xaa₁₀ is Ser, or Thr, and Xaa₁₁ is Val.

In some embodiments, Xaa₁₂-Xaa₁₃ is Leu-Leu. Further, Xaa₁₄ is Thr, or Ser, Xaa₁₅ is Ser, or Thr, and Xaa₁₆ is Val.

In some embodiments, Xaa₁₇-Xaa₁₈-Xaa₁₉ is Asp-Lys-Ala, Xaa₂₀ is Thr, or Ser, and Xaa₂₁ is Ser, or Thr.

In some embodiments, Xaa₂₂-Xaa₂₃ is Leu-Leu. Further, Xaa₂₄ is Thr, or Ser, Xaa₂₅ is Ser, or Thr, and Xaa₂₆ is Val.

Each of the CCR8 antagonistic polypeptide compounds and variants thereof herein retains the ability of binding to CCR8 and blocking the CCL1-CCR8 signaling pathway.

In some embodiments, the present application provides a polynucleotide, encoding the CCR8 antagonistic polypeptide compound.

It is known by those skilled in the art that due to codon degeneracy, each amino acid sequence can be encoded by several nucleic acid sequences. The nucleic acid sequence encoding the polypeptide of the present disclosure can be synthesized using methods well known in the art (such as *de novo* solid-phase DNA synthesis, PCR amplification).

The polypeptide of the present disclosure is preferably synthesized by using a solid-phase synthesis method.

### Pharmaceutical Composition and Kit

In the second aspect, the present application provides a pharmaceutical composition, comprising the polypeptide compound and a pharmaceutically acceptable excipient. Alternatively, the present application provides a kit, comprising the polypeptide compound or the pharmaceutical composition thereof.

In some embodiments, the pharmaceutically acceptable excipient may be selected from, for example, but is not limited to, solvents, propellants, solubilizers, cosolvents, emulsifiers, colorants, disintegrants, fillers, lubricants, wetting agents, osmotic pressure regulators, stabilizers, glidants, flavoring agents, preservatives, suspending agents, antioxidants, penetration enhancers, pH adjusters, surfactants, diluents, etc. Regarding other available pharmaceutically acceptable excipients, it can be found, for example, in R.C. Rowe *et al.*, translated mainly by Junmin Zheng, Handbook of Pharmaceutical Excipients, 4th ed., Chemical Industry Press, 2005.

In some embodiments, the pharmaceutical composition further comprises additional therapeutic agent, including, but not limited to, glucocorticoids, anti-inflammatory drugs, anti-pruritic drugs, antiviral drugs, antihistamines, immunosuppressants, antipyretics, anticancer drugs, antifibrotic drugs, etc.

In some embodiments, the pharmaceutical composition further comprises an additional therapeutic agent for atopic dermatitis, such as glucocorticoids (e.g., hydrocortisone, hydrocortisone butyrate, triamcinolone acetonide, mometasone, mometasone furoate, etc.), calcineurin inhibitors (e.g., tacrolimus, pimecrolimus), PDE4 inhibitors (e.g., crisaborole, roflumilast), JAK inhibitors (e.g., ruxolitinib, upadacitinib), antibiotics (e.g., mupirocin, fusidic acid, erythromycin, quinolone antibiotics (including norfloxacin, ofloxacin, pefloxacin, enoxacin, ciprofloxacin, fleroxacin, gatifloxacin, moxifloxacin), tetracyclines), anti-pruritic drugs (e.g., doxepin, flurbiprofen, diclofenac diethylamine, diclofenac, ibuprofen), wet compress drugs (e.g., aluminum acetate solution, potassium permanganate solution), calamine lotion or zinc oxide cream, antihistamines (e.g., loratadine, desloratadine, mizolastine, ebastine), antiviral drugs (e.g., acyclovir, valacyclovir, penciclovir), immunosuppressants (e.g., cyclosporine, azathioprine, methotrexate), etc.

In some embodiments, the pharmaceutical composition of the present disclosure may be powder, granule, film-coating agent, cream, ointment, gel, lotion, foam, patch, liniment, elixir, aerosol, emulsion, solution or suspension.

In some embodiments, the kit may further comprise an instruction for use. In some embodiments, the kit may further comprise reagents for diagnosing a patient.

In some embodiments, the kit also comprises tools that can be used for administering the drug, such as syringes, catheters, swabs, cotton swabs, etc.

### Use

In the third aspect of the present disclosure, the present application provides use of the polypeptide compound according to the first aspect, or the pharmaceutical composition or kit according to the second aspect of the present disclosure in the preparation of a medicament for preventing or treating cancers or tumors, inflammatory diseases, viral infections, IgG4-related diseases, cutaneous immune diseases, or related symptoms thereof.

In some embodiments, the present application relates to a method of preventing or treating cancers or tumors, inflammatory diseases, viral infections, IgG4-related diseases, cutaneous immune diseases, or related symptoms thereof in a subject in need thereof, comprising administering to the subject a therapeutically effective amount of the polypeptide compound according to the first aspect, or the pharmaceutical composition or kit according to the second aspect of the present disclosure.

In some embodiments, the present application relates to the polypeptide compound according to the first aspect, or the pharmaceutical composition or kit according to the second aspect of the present disclosure for use in preventing or treating cancers or tumors, inflammatory diseases, viral infections, IgG4-related diseases, cutaneous immune diseases, or related symptoms thereof.

In some embodiments, the present application relates to use of the polypeptide compound according to the first aspect, or the pharmaceutical composition or kit according to the second aspect of the present disclosure in the preparation of an agent for blocking the CCL1-CCR8 signaling pathway.

In some embodiments, the present application relates to a method of blocking the CCL1-CCR8 signaling pathway in a subject in need thereof, comprising administering to the subject an effective amount of the polypeptide compound according to the first aspect, or the pharmaceutical composition or kit according to the second aspect of the present disclosure.

In some embodiments, the present application relates to the polypeptide compound according to the first aspect, or the pharmaceutical composition or kit according to the second aspect of the present disclosure for use in blocking the CCL1-CCR8 signaling pathway.

In some embodiments, the tumor is selected from solid tumors, more preferably multiple myeloma, lymphoma or melanoma; the cancer is selected from esophageal cancer, lung cancer, non-small cell lung cancer, gastric cancer, head and neck squamous cell carcinoma, liver cancer, breast cancer, cervical cancer, ovarian cancer, colorectal cancer, colon cancer, rectal cancer, kidney cancer, bladder cancer, dermatofibrosarcoma, osteosarcoma, pancreatic cancer, and prostate cancer.

In some embodiments, the inflammatory disease is selected from asthma and allergic enteritis.

In some embodiments, the IgG4-related disease is selected from sialadenitis.

In some embodiments, the cutaneous immune disease or related symptoms thereof are cutaneous immune diseases related to the CCL1-CCR8 signaling pathway or related symptoms thereof.

In some embodiments, the cutaneous immune disease or related symptoms thereof are selected from eczema, atopic dermatitis, psoriasis, lupus erythematosus, scleroderma, dermatomyositis, pemphigus, bullous pemphigoid, dermatitis herpetiformis, epidermolysis bullosa, and related symptoms thereof. In some preferred embodiments, the cutaneous immune disease or related symptoms thereof are selected from atopic dermatitis, and related symptoms thereof (e.g., dry skin, skin pruritus, eczema-like skin lesions (including erythema, papules, redness and swelling, blisters, skin erosion, epidermal thickening), xeroderma-related symptoms, hyperlinear palms-related symptoms, white dermatographism, Hertoghe's sign, infraorbital fold, periorbital darkening (dark circles under the eyes), low posterior hairline). Preferably, the atopic dermatitis is infantile atopic dermatitis, childhood atopic dermatitis, or adolescent and adult atopic dermatitis. Or preferably, the atopic dermatitis is mild, moderate or severe atopic dermatitis. Various methods for evaluating the severity of atopic dermatitis are known at present. For example, the following indicators can be used clinically to determine the severity of atopic dermatitis: mild atopic dermatitis has a rash area of less than 5%; moderate atopic dermatitis has a rash area of 5%-10%, or the rash occurs repeatedly; and severe atopic dermatitis has skin lesions exceeding 10% of the body surface area, or the dermatitis is persistent with severe itching affecting sleep.

In some embodiments, the pharmaceutical composition may be in the form of a sterile aqueous solution, microemulsion, liposome or powder.

In some embodiments, the pharmaceutical composition may be in the form of a unit dose to facilitate administration to a patient at a desired dosage.

Herein, "therapeutically effective amount" or "effective amount" refers to an amount capable of producing a desired response (including preventing or slowing disease onset, reducing disease severity, improving patient status, inducing immune response, delaying or at least slowing disease progression, or curing) in a subject or patient in need thereof, which can be determined by those skilled in the art based on the subject or patient's age, gender, weight, physical condition, family history, risk of onset, sensitivity to drugs, etc. in combination with the contents of the present disclosure. The effective amount of administration can be administered to a subject in a single dose, or by dividing it into multiple identical or different doses. The dosage range of the pharmaceutical composition herein can be determined empirically by a clinician based on the administration mode (including administration time, administration interval, route of administration), the patient's age, weight, gender or pathological condition, diet, excretion rate, and sensitivity to the drug, etc.

The polypeptide, pharmaceutical composition or kit can be administered (e.g., by injection or infusion) to a subject in need thereof via conventional administration routes in the art (e.g., parenteral, intravenous, intramuscular, intraarterial, intraperitoneal, subcutaneous, transdermal, topical administration). The polypeptide or pharmaceutical composition of the present application can be formulated into forms such as injection, sterile aqueous solution, emulsion, suspension, liposome, gel or powder.

The CCR8 antagonistic polypeptide compound herein can exhibit excellent ability of blocking the CCL1-CCR8 signaling pathway, and thus can be expected to show good therapeutic effects on diseases involving the CCL1-CCR8 signaling pathway and related symptoms thereof (e.g., atopic dermatitis) in tested animals *in vivo*.

### EXAMPLE

Next, the present application will be further described in details through examples, but the protection scope of the present application is not limited thereto.

Unless otherwise specified, the reagents, materials, and instruments used in the following examples are reagents, materials, and instruments conventionally used in the art, which are commercially available or can be synthesized by conventional methods known in the art.

### Example 1: Composition and Synthesis of Polypeptide Compounds

The polypeptide compounds having an inhibitory function on chemokine receptor 8 (CCR8) used in the following Examples and Experimental Examples comprise the amino acid sequence set forth in any one of SEQ ID NO: 7 to SEQ ID NO: 205, shown as Nos. TCR01-TCR153 and TCR155-TCR200, respectively, as specifically listed in the table below.

| **Compound No.** | **Composition of Sequence** | **SEQ ID NO** |
|---|---|---|
| TCR01 | H₂N-AIB-Arg-Pro-Asp-Lys-Ala-Ala-Leu-Gly-Tyr-Gln-Lys-Arg-Pro-Leu-Pro-Gln-Val-Leu-Leu-Thr-Thr-Leu-CONH₂ | 7 |
| TCR02 | H₂N-AIB-Arg-Pro-Asp-Lys-Ala-Ala-Leu-Gly-Tyr-Gln-Lys-Arg-Pro-Leu-Pro-Gln-Val-Leu-Leu-Ala-Ala-Leu-CONH₂ | 8 |
| TCR03 | H₂N-AIB-Arg-Pro-Asp-Lys-Ala-Ala-Leu-Gly-Tyr-Gln-Lys-Arg-Pro-Leu-Pro-Gln-Val-Leu-Leu-Ala-Thr-Leu-CONH₂ | 9 |
| TCR04 | H₂N-AIB-Arg-Pro-Asp-Lys-Ala-Ala-Leu-Gly-Tyr-Gln-Lys-Arg-Pro-Leu-Pro-Gln-Val-Leu-Leu-Thr-Ala-Leu-CONH₂ | 10 |
| TCR05 | H₂N-AIB-Arg-Pro-Asp-Lys-Ala-Ala-Leu-Gly-Tyr-Gln-Lys-Arg-Pro-Leu-Pro-Gln-Val-Leu-Leu-Thr-Thr-Val-CONH₂ | 11 |
| TCR06 | H₂N-AIB-Arg-Pro-Asp-Lys-Ala-Ala-Leu-Gly-Tyr-Gln-Lys-Arg-Pro-Leu-Pro-Gln-Val-Leu-Leu-Thr-Ala-Val-CONH₂ | 12 |
| TCR07 | H₂N-AIB-Arg-Pro-Asp-Lys-Ala-Ala-Leu-Gly-Tyr-Gln-Lys-Arg-Pro-Leu-Pro-Gln-Val-Leu-Leu-Ala-Thr-Val-CONH₂ | 13 |
| TCR08 | H₂N-AIB-Arg-Pro-Asp-Lys-Ala-Ala-Leu-Gly-Tyr-Gln-Lys-Arg-Pro-Leu-Pro-Gln-Val-Leu-Leu-Ser-Thr-Val-CONH₂ | 14 |
| TCR09 | H₂N-AIB-Arg-Pro-Asp-Lys-Ala-Ala-Leu-Gly-Tyr-Gln-Lys-Arg-Pro-Leu-Pro-Gln-Val-Leu-Leu-Ser-Ser-Val-CONH₂ | 15 |
| TCR10 | H₂N-AIB-Arg-Pro-Asp-Lys-Ala-Ala-Leu-Gly-Tyr-Gln-Lys-Arg-Pro-Leu-Pro-Gln-Val-Leu-Leu-Ser-Ser-Ile-CONH₂ | 16 |
| TCR11 | H₂N-AIB-Arg-Pro-Asp-Lys-Ala-Ala-Leu-Gly-Tyr-Gln-Lys-Arg-Pro-Leu-Pro-Gln-Val-Leu-Leu-Thr-Ser-AIB-CONH₂ | 17 |
| TCR12 | H₂N-AIB-Arg-Pro-Asp-Lys-Ala-Ala-Leu-Gly-Tyr-Gln-Lys-Arg-Pro-Leu-Pro-Gln-Val-Leu-Leu-Ser-Ser-AIB-CONH₂ | 18 |
| TCR13 | H₂N-AIB-Arg-Pro-Asp-Lys-Ala-Ala-Leu-Gly-Tyr-Gln-Lys-Arg-Pro-Leu-Pro-Gln-Val-Leu-Leu-Ser-Thr-AIB-CONH₂ | 19 |
| TCR14 | H₂N-AIB-Arg-Pro-Asp-Lys-Ala-Ala-Leu-Gly-Tyr-Gln-Lys-Arg-Pro-Leu-Pro-Gln-Val-Leu-Leu-Ala-Thr-AIB-CONH₂ | 20 |
| TCR15 | H₂N-AIB-Arg-Pro-Asp-Lys-Ala-Ala-Leu-Gly-Tyr-Gln-Lys-Arg-Pro-Leu-Pro-Gln-Val-Leu-Leu-Thr-Thr-AIB-CONH₂ | 21 |
| TCR16 | H₂N-AIB-Arg-Pro-Asp-Lys-Ala-Ala-Leu-Gly-Tyr-Gln-Lys-Arg-Pro-Leu-Pro-Gln-Val-Leu-Leu-Thr-Thr-Cys-CONH₂ | 22 |
| TCR17 | H₂N-AIB-Arg-Pro-Asp-Lys-Ala-Ala-Leu-Gly-Tyr-Gln-Lys-Arg-Pro-Leu-Pro-Gln-Val-Leu-Leu-Thr-Ser-Cys-CONH₂ | 23 |
| TCR18 | H₂N-AIB-Arg-Pro-Asp-Lys-Ala-Ala-Leu-Gly-Tyr-Gln-Lys-Arg-Pro-Leu-Pro-Gln-Val-Leu-Leu-Ser-Ser-Cys-CONH₂ | 24 |
| TCR19 | H₂N-AIB-Arg-Pro-Asp-Lys-Ala-Ala-Leu-Gly-Tyr-Gln-Lys-Arg-Pro-Leu-Pro-Gln-Val-Leu-Leu-Ser-Thr-Cys-CONH₂ | 25 |
| TCR20 | H₂N-AIB-Arg-Pro-Asp-Lys-Ala-Ala-Leu-Gly-Tyr-Gln-Lys-Arg-Pro-Leu-Pro-Gln-Val-Leu-Leu-Ala-Thr-Cys-CONH₂ | 26 |
| TCR21 | H₂N-AIB-Arg-Pro-Asp-Lys-Ala-Ala-Leu-Gly-Tyr-Gln-Lys-Arg-Pro-Leu-Pro-Gln-Val-Leu-Leu-Thr-Thr-Nle-CONH₂ | 27 |
| TCR22 | H₂N-AIB-Arg-Pro-Asp-Lys-Ala-Ala-Leu-Gly-Tyr-Gln-Lys-Arg-Pro-Leu-Pro-Gln-Val-Leu-Leu-Thr-Ser-Nle-CONH₂ | 28 |
| TCR23 | H₂N-AIB-Arg-Pro-Asp-Lys-Ala-Ala-Leu-Gly-Tyr-Gln-Lys-Arg-Pro-Leu-Pro-Gln-Val-Leu-Leu-Ser-Ser-Nle-CONH₂ | 29 |
| TCR24 | H₂N-AIB-Arg-Pro-Asp-Lys-Ala-Ala-Leu-Gly-Tyr-Gln-Lys-Arg-Pro-Leu-Pro-Gln-Val-Leu-Leu-Ser-Thr-Nle-CONH₂ | 30 |
| TCR25 | H₂N-AIB-Arg-Pro-Asp-Lys-Ala-Ala-Leu-Gly-Tyr-Gln-Lys-Arg-Pro-Leu-Pro-Gln-Val-Leu-Leu-Ala-Thr-Nle-CONH₂ | 31 |
| TCR26 | H₂N-AIB-Arg-Pro-Asp-Lys-Ala-Ala-Leu-Gly-Tyr-Gln-Lys-Arg-Pro-Leu-Pro-Gln-Val-Leu-Leu-Thr-Thr-Ala-CONH₂ | 32 |
| TCR27 | H₂N-AIB-Arg-Pro-Asp-Lys-Ala-Ala-Leu-Gly-Tyr-Gln-Lys-Arg-Pro-Leu-Pro-Gln-Val-Leu-Leu-Thr-Ser-Ala-CONH₂ | 33 |
| TCR28 | H₂N-AIB-Arg-Pro-Asp-Lys-Ala-Ala-Leu-Gly-Tyr-Gln-Lys-Arg-Pro-Leu-Pro-Gln-Val-Leu-Leu-Ser-Ser-Ala-CONH₂ | 34 |
| TCR29 | H₂N-AIB-Arg-Pro-Asp-Lys-Ala-Ala-Leu-Gly-Tyr-Gln-Lys-Arg-Pro-Leu-Pro-Gln-Val-Leu-Leu-Ser-Thr-Ala-CONH₂ | 35 |
| TCR30 | H₂N-AIB-Arg-Pro-Asp-Lys-Ala-Ala-Leu-Gly-Tyr-Gln-Lys-Arg-Pro-Leu-Pro-Gln-Val-Leu-Leu-Ala-Thr-Ala-CONH₂ | 36 |
| TCR31 | H₂N-AIB-Arg-Pro-Asp-Lys-Ala-Ala-Leu-Gly-Tyr-Gln-Lys-Arg-Pro-Leu-Pro-Gln-Val-Leu-Leu-Thr-Thr-Met-CONH₂ | 37 |
| TCR32 | H₂N-AIB-Arg-Pro-Asp-Lys-Ala-Ala-Leu-Gly-Tyr-Gln-Lys-Arg-Pro-Leu-Pro-Gln-Val-Leu-Leu-Thr-Ser-Met-CONH₂ | 38 |
| TCR33 | H₂N-AIB-Arg-Pro-Asp-Lys-Ala-Ala-Leu-Gly-Tyr-Gln-Lys-Arg-Pro-Leu-Pro-Gln-Val-Leu-Leu-Ser-Ser-Met-CONH₂ | 39 |
| TCR34 | H₂N-AIB-Arg-Pro-Asp-Lys-Ala-Ala-Leu-Gly-Tyr-Gln-Lys-Arg-Pro-Leu-Pro-Gln-Val-Leu-Leu-Ser-Thr-Met-CONH₂ | 40 |
| TCR35 | H₂N-AIB-Arg-Pro-Asp-Lys-Ala-Ala-Leu-Gly-Tyr-Gln-Lys-Arg-Pro-Leu-Pro-Gln-Val-Leu-Leu-Ala-Thr-Met-CONH₂ | 41 |
| TCR36 | H₂N-AIB-Arg-Pro-Asp-Lys-Ala-Ala-Leu-Gly-Tyr-Gln-Lys-Arg-Pro-Leu-Pro-Gln-Val-Leu-Leu-Thr-Thr-Leu-CONH₂ | 42 |
| TCR37 | H₂N-AIB-Arg-Pro-Asp-Lys-Ala-Ala-Leu-Gly-Tyr-Gln-Lys-Arg-Pro-Leu-Pro-Gln-Val-Val-Val-Thr-Thr-Leu-CONH₂ | 43 |
| TCR38 | H₂N-AIB-Arg-Pro-Asp-Lys-Ala-Ala-Leu-Gly-Tyr-Gln-Lys-Arg-Pro-Leu-Pro-Gln-Val-Val-Leu-Thr-Thr-Leu-CONH₂ | 44 |
| TCR39 | H₂N-AIB-Arg-Pro-Asp-Lys-Ala-Ala-Leu-Gly-Tyr-Gln-Lys-Arg-Pro-Leu-Pro-Gln-Val-Leu-Val-Thr-Thr-Leu-CONH₂ | 45 |
| TCR40 | H₂N-AIB-Arg-Pro-Asp-Lys-Ala-Ala-Leu-Gly-Tyr-Gln-Lys-Arg-Pro-Leu-Pro-Gln-Val-Leu-Ile-Thr-Thr-Leu-CONH₂ | 46 |
| TCR41 | H₂N-AIB-Arg-Pro-Asp-Lys-Ala-Ala-Leu-Gly-Tyr-Gln-Lys-Arg-Pro-Leu-Pro-Gln-Val-Leu-Ile-Ile-Thr-Leu-CONH₂ | 47 |
| TCR42 | H₂N-AIB-Arg-Pro-Asp-Lys-Ala-Ala-Leu-Gly-Tyr-Gln-Lys-Arg-Pro-Leu-Pro-Gln-Val-Val-Ile-Thr-Thr-Leu-CONH₂ | 48 |
| TCR43 | H₂N-AIB-Arg-Pro-Asp-Lys-Ala-Ala-Leu-Gly-Tyr-Gln-Lys-Arg-Pro-Leu-Pro-Gln-Ala-Val-Leu-Thr-Thr-Leu-CONH₂ | 49 |
| TCR44 | H₂N-AIB-Arg-Pro-Asp-Lys-Ala-Ala-Leu-Gly-Tyr-Gln-Lys-Arg-Pro-Leu-Pro-Gln-Leu-Leu-Val-Thr-Thr-Leu-CONH₂ | 50 |
| TCR45 | H₂N-AIB-Arg-Pro-Asp-Lys-Ala-Ala-Leu-Gly-Tyr-Gln-Lys-Arg-Pro-Leu-Pro-Gln-Ile-Leu-Ile-Thr-Thr-Leu-CONH₂ | 51 |
| TCR46 | H₂N-AIB-Arg-Pro-Asp-Lys-Ala-Ala-Leu-Gly-Tyr-Gln-Lys-Arg-Pro-Leu-Pro-Gln-Ala-Val-Ile-Thr-Thr-AIB-CONH₂ | 52 |
| TCR47 | H₂N-AIB-Arg-Pro-Asp-Lys-Ala-Ala-Leu-Gly-Tyr-Gln-Lys-Arg-Pro-Leu-Pro-Gln-Val-Val-Ile-Thr-Thr-Leu-CONH₂ | 53 |
| TCR48 | H₂N-AIB-Arg-Pro-Asp-Lys-Ala-Ala-Leu-Gly-Tyr-Gln-Lys-Arg-Pro-Leu-Pro-Gln-Ala-Val-Ile-Thr-Ser-AIB-CONH₂ | 54 |
| TCR49 | H₂N-AIB-Arg-Pro-Asp-Lys-Ala-Ala-Leu-Gly-Tyr-Gln-Lys-Arg-Pro-Leu-Pro-Gln-Leu-Leu-Val-Thr-Thr-AIB-CONH₂ | 55 |
| TCR50 | H₂N-AIB-Arg-Pro-Asp-Lys-Ala-Ala-Leu-Gly-Tyr-Gln-Lys-Arg-Pro-Leu-Pro-Gln-Ile-Leu-Ile-Thr-Thr-AIB-CONH₂ | 56 |
| TCR51 | H₂N-AIB-Arg-Pro-Asp-Lys-Ala-Ala-Leu-Gly-Tyr-Gln-Lys-Arg-Pro-Ile-Pro-Asn-Ile-Leu-Leu-Thr-Thr-Leu-CONH₂ | 57 |
| TCR52 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ala-Ala-Leu-CONH₂ | 58 |
| TCR53 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ala-Thr-Leu-CONH₂ | 59 |
| TCR54 | H₂N-AIB-Arg-Pro-Asp-Lys-Ala-Ala-Leu-Gly-Tyr-Gln-Lys-Arg-Pro-Ile-Pro-Asn-Ile-Leu-Leu-Thr-Ala-Leu-CONH₂ | 60 |
| TCR55 | H₂N-AIB-Arg-Pro-Asp-Lys-Ala-Ala-Leu-Gly-Tyr-Gln-Lys-Arg-Pro-Ile-Pro-Asn-Ile-Leu-Leu-Thr-Thr-Val-CONH₂ | 61 |
| TCR56 | H₂N-AIB-Arg-Pro-Asp-Lys-Ala-Ala-Leu-Gly-Tyr-Gln-Lys-Arg-Pro-Ile-Pro-Asn-Ile-Leu-Leu-Thr-Ala-Val-CONH₂ | 62 |
| TCR57 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ala-Thr-Val-CONH₂ | 63 |
| TCR58 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ser-Thr-Val-CONH₂ | 64 |
| TCR59 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ser-Ser-Val-CONH₂ | 65 |
| TCR60 | H₂N-AIB-Arg-Pro-Asp-Lys-Ala-Ala-Leu-Gly-Tyr-Gln-Lys-Arg-Pro-Ile-Pro-Asn-Ile-Leu-Leu-Thr-Ser-Val-CONH₂ | 66 |
| TCR61 | H₂N-AIB-Arg-Pro-Asp-Lys-Ala-Ala-Leu-Gly-Tyr-Gln-Lys-Arg-Pro-Ile-Pro-Asn-Ile-Leu-Leu-Thr-Ser-AIB-CONH₂ | 67 |
| TCR62 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ser-Ser-AIB-CONH₂ | 68 |
| TCR63 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ser-Thr-AIB-CONH₂ | 69 |
| TCR64 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ala-Thr-AIB-CONH₂ | 70 |
| TCR65 | H₂N-AIB-Arg-Pro-Asp-Lys-Ala-Ala-Leu-Gly-Tyr-Gln-Lys-Arg-Pro-Ile-Pro-Asn-Ile-Leu-Leu-Thr-Thr-AIB-CONH₂ | 71 |
| TCR66 | H₂N-AIB-Arg-Pro-Asp-Lys-Ala-Ala-Leu-Gly-Tyr-Gln-Lys-Arg-Pro-Ile-Pro-Asn-Ile-Leu-Leu-Thr-Thr-Cys-CONH₂ | 72 |
| TCR67 | H₂N-AIB-Arg-Pro-Asp-Lys-Ala-Ala-Leu-Gly-Tyr-Gln-Lys-Arg-Pro-Ile-Pro-Asn-Ile-Leu-Leu-Thr-Ser-Cys-CONH₂ | 73 |
| TCR68 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ser-Ser-Cys-CONH₂ | 74 |
| TCR69 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ser-Thr-Cys-CONH₂ | 75 |
| TCR70 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ala-Thr-Cys-CONH₂ | 76 |
| TCR71 | H₂N-AIB-Arg-Pro-Asp-Lys-Ala-Ala-Leu-Gly-Tyr-Gln-Lys-Arg-Pro-Ile-Pro-Asn-Ile-Leu-Leu-Thr-Thr-Nle-CONH₂ | 77 |
| TCR72 | H₂N-AIB-Arg-Pro-Asp-Lys-Ala-Ala-Leu-Gly-Tyr-Gln-Lys-Arg-Pro-Ile-Pro-Asn-Ile-Leu-Leu-Thr-Ser-Nle-CONH₂ | 78 |
| TCR73 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ser-Ser-Nle-CONH₂ | 79 |
| TCR74 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ser-Thr-Nle-CONH₂ | 80 |
| TCR75 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ala-Thr-Nle-CONH₂ | 81 |
| TCR76 | H₂N-AIB-Arg-Pro-Asp-Lys-Ala-Ala-Leu-Gly-Tyr-Gln-Lys-Arg-Pro-Ile-Pro-Asn-Ile-Leu-Leu-Thr-Thr-Ala-CONH₂ | 82 |
| TCR77 | H₂N-AIB-Arg-Pro-Asp-Lys-Ala-Ala-Leu-Gly-Tyr-Gln-Lys-Arg-Pro-Ile-Pro-Asn-Ile-Leu-Leu-Thr-Ser-Ala-CONH₂ | 83 |
| TCR78 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ser-Ser-Ala-CONH₂ | 84 |
| TCR79 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ser-Thr-Ala-CONH₂ | 85 |
| TCR80 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ala-Thr-Ala-CONH₂ | 86 |
| TCR81 | H₂N-AIB-Arg-Pro-Asp-Lys-Ala-Ala-Leu-Gly-Tyr-Gln-Lys-Arg-Pro-Ile-Pro-Asn-Ile-Leu-Leu-Thr-Thr-Met-CONH₂ | 87 |
| TCR82 | H₂N-AIB-Arg-Pro-Asp-Lys-Ala-Ala-Leu-Gly-Tyr-Gln-Lys-Arg-Pro-Ile-Pro-Asn-Ile-Leu-Leu-Thr-Ser-Met-CONH₂ | 88 |
| TCR83 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ser-Ser-Met-CONH₂ | 89 |
| TCR84 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ser-Thr-Met-CONH₂ | 90 |
| TCR85 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ala-Thr-Met-CONH₂ | 91 |
| TCR86 | H₂N-AIB-Arg-Pro-Asp-Lys-Ala-Ala-Leu-Gly-Tyr-Gln-Lys-Arg-Pro-Ile-Pro-Asn-Ile-Leu-Leu-Thr-Thr-Leu-CONH₂ | 92 |
| TCR87 | H₂N-AIB-Arg-Pro-Asp-Lys-Ala-Ala-Leu-Gly-Tyr-Gln-Lys-Arg-Pro-Ile-Pro-Asn-Ile-Val-Val-Thr-Thr-Leu-CONH₂ | 93 |
| TCR88 | H₂N-AIB-Arg-Pro-Asp-Lys-Ala-Ala-Leu-Gly-Tyr-Gln-Lys-Arg-Pro-Ile-Pro-Asn-Ile-Val-Leu-Thr-Thr-Leu-CONH₂ | 94 |
| TCR89 | H₂N-AIB-Arg-Pro-Asp-Lys-Ala-Ala-Leu-Gly-Tyr-Gln-Lys-Arg-Pro-Ile-Pro-Asn-Ile-Leu-Val-Thr-Thr-Leu-CONH₂ | 95 |
| TCR90 | H₂N-AIB-Arg-Pro-Asp-Lys-Ala-Ala-Leu-Gly-Tyr-Gln-Lys-Arg-Pro-Ile-Pro-Asn-Ile-Leu-Ile-Thr-Thr-Leu-CONH₂ | 96 |
| TCR91 | H₂N-AIB-Arg-Pro-Asp-Lys-Ala-Ala-Leu-Gly-Tyr-Gln-Lys-Arg-Pro-Leu-Pro-Gln-Val-Leu-Leu-Thr-Thr-Abu-CONH₂ | 97 |
| TCR92 | H₂N-AIB-Arg-Pro-Asp-Lys-Ala-Ala-Leu-Gly-Tyr-Gln-Lys-Arg-Pro-Leu-Pro-Gln-Val-Leu-Leu-Thr-Ser-Abu-CONH₂ | 98 |
| TCR93 | H₂N-AIB-Arg-Pro-Asp-Lys-Ala-Ala-Leu-Gly-Tyr-Gln-Lys-Arg-Pro-Leu-Pro-Gln-Val-Leu-Leu-Ser-Ser-Abu-CONH₂ | 99 |
| TCR94 | H₂N-AIB-Arg-Pro-Asp-Lys-Ala-Ala-Leu-Gly-Tyr-Gln-Lys-Arg-Pro-Leu-Pro-Gln-Val-Leu-Leu-Ser-Thr-Abu-CONH₂ | 100 |
| TCR95 | H₂N-AIB-Arg-Pro-Asp-Lys-Ala-Ala-Leu-Gly-Tyr-Gln-Lys-Arg-Pro-Leu-Pro-Gln-Val-Leu-Leu-Ala-Thr-Abu-CONH₂ | 101 |
| TCR96 | H₂N-AIB-Arg-Pro-Asp-Lys-Ala-Ala-Leu-Gly-Tyr-Gln-Lys-Arg-Pro-Ile-Pro-Asn-Ile-Leu-Leu-Thr-Thr-Abu-CONH₂ | 102 |
| TCR97 | H₂N-AIB-Arg-Pro-Asp-Lys-Ala-Ala-Leu-Gly-Tyr-Gln-Lys-Arg-Pro-Ile-Pro-Asn-Ile-Leu-Leu-Thr-Ser-Abu-CONH₂ | 103 |
| TCR98 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ser-Ser-Abu-CONH₂ | 104 |
| TCR99 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ser-Thr-Abu-CONH₂ | 105 |
| TCR100 | H₂N-AIB-Arg-Pro-Asp-Lys-Ala-Ala-Leu-Gly-Tyr-Gln-Lys-Arg-Pro-Ile-Pro-Asn-Ile-Leu-Leu-Ala-Thr-Abu-CONH₂ | 106 |
| TCR101 | H₂N-AIB-Arg-Pro-Asp-Lys-Ala-Ala-Leu-Gly-Tyr-Asn-Orn-Arg-Pro-Ile-Pro-Asn-Ile-Leu-Leu-Thr-Thr-Leu-CONH₂ | 107 |
| TCR102 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ala-Ala-Leu-CONH₂ | 108 |
| TCR103 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ala-Thr-Leu-CONH₂ | 109 |
| TCR104 | H₂N-AIB-Arg-Pro-Asp-Lys-Ala-Ala-Leu-Gly-Tyr-Asn-Orn-Arg-Pro-Ile-Pro-Asn-Ile-Leu-Leu-Thr-Ala-Leu-CONH₂ | 110 |
| TCR105 | H₂N-AIB-Arg-Pro-Asp-Lys-Ala-Ala-Leu-Gly-Tyr-Asn-Orn-Arg-Pro-Ile-Pro-Asn-Ile-Leu-Leu-Thr-Thr-Val-CONH₂ | 111 |
| TCR106 | H₂N-AIB-Arg-Pro-Asp-Lys-Ala-Ala-Leu-Gly-Tyr-Asn-Orn-Arg-Pro-Ile-Pro-Asn-Ile-Leu-Leu-Thr-Ala-Val-CONH₂ | 112 |
| TCR107 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ala-Thr-Val-CONH₂ | 113 |
| TCR108 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ser-Thr-Val-CONH₂ | 114 |
| TCR109 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ser-Ser-Val-CONH₂ | 115 |
| TCR110 | H₂N-AIB-Arg-Pro-Asp-Lys-Ala-Ala-Leu-Gly-Tyr-Asn-Orn-Arg-Pro-Ile-Pro-Asn-Ile-Leu-Leu-Thr-Ser-Val-CONH₂ | 116 |
| TCR111 | H₂N-AIB-Arg-Pro-Asp-Lys-Ala-Ala-Leu-Gly-Tyr-Asn-Orn-Arg-Pro-Ile-Pro-Asn-Ile-Leu-Leu-Thr-Ser-AIB-CONH₂ | 117 |
| TCR112 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ser-Ser-AIB-CONH₂ | 118 |
| TCR113 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ser-Thr-AIB-CONH₂ | 119 |
| TCR114 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ala-Thr-AIB-CONH₂ | 120 |
| TCR115 | H₂N-AIB-Arg-Pro-Asp-Lys-Ala-Ala-Leu-Gly-Tyr-Asn-Orn-Arg-Pro-Ile-Pro-Asn-Ile-Leu-Leu-Thr-Thr-AIB-CONH₂ | 121 |
| TCR116 | H₂N-AIB-Arg-Pro-Asp-Lys-Ala-Ala-Leu-Gly-Tyr-Asn-Orn-Arg-Pro-Ile-Pro-Asn-Ile-Leu-Leu-Thr-Thr-Cys-CONH₂ | 122 |
| TCR117 | H₂N-AIB-Arg-Pro-Asp-Lys-Ala-Ala-Leu-Gly-Tyr-Asn-Orn-Arg-Pro-Ile-Pro-Asn-Ile-Leu-Leu-Thr-Ser-Cys-CONH₂ | 123 |
| TCR118 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ser-Ser-Cys-CONH₂ | 124 |
| TCR119 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ser-Thr-Cys-CONH₂ | 125 |
| TCR120 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ala-Thr-Cys-CONH₂ | 126 |
| TCR121 | H₂N-AIB-Arg-Pro-Asp-Lys-Ala-Ala-Leu-Gly-Tyr-Asn-Orn-Arg-Pro-Ile-Pro-Asn-Ile-Leu-Leu-Thr-Thr-Nle-CONH₂ | 127 |
| TCR122 | H₂N-AIB-Arg-Pro-Asp-Lys-Ala-Ala-Leu-Gly-Tyr-Asn-Orn-Arg-Pro-Ile-Pro-Asn-Ile-Leu-Leu-Thr-Ser-Nle-CONH₂ | 128 |
| TCR123 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ser-Ser-Nle-CONH₂ | 129 |
| TCR124 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ser-Thr-Nle-CONH₂ | 130 |
| TCR125 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ala-Thr-Nle-CONH₂ | 131 |
| TCR126 | H₂N-AIB-Arg-Pro-Asp-Lys-Ala-Ala-Leu-Gly-Tyr-Asn-Orn-Arg-Pro-Ile-Pro-Asn-Ile-Leu-Leu-Thr-Thr-Ala-CONH₂ | 132 |
| TCR127 | H₂N-AIB-Arg-Pro-Asp-Lys-Ala-Ala-Leu-Gly-Tyr-Asn-Orn-Arg-Pro-Ile-Pro-Asn-Ile-Leu-Leu-Thr-Ser-Ala-CONH₂ | 133 |
| TCR128 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ser-Ser-Ala-CONH₂ | 134 |
| TCR129 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ser-Thr-Ala-CONH₂ | 135 |
| TCR130 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ala-Thr-Ala-CONH₂ | 136 |
| TCR131 | H₂N-AIB-Arg-Pro-Asp-Lys-Ala-Ala-Leu-Gly-Tyr-Asn-Orn-Arg-Pro-Ile-Pro-Asn-Ile-Leu-Leu-Thr-Thr-Leu-CONH₂ | 137 |
| TCR132 | H₂N-AIB-Arg-Pro-Asp-Lys-Ala-Ala-Leu-Gly-Tyr-Asn-Orn-Arg-Pro-Ile-Pro-Asn-Ile-Val-Val-Thr-Thr-Leu-CONH₂ | 138 |
| TCR133 | H₂N-AIB-Arg-Pro-Asp-Lys-Ala-Ala-Leu-Gly-Tyr-Asn-Orn-Arg-Pro-Ile-Pro-Asn-Ile-Val-Leu-Thr-Thr-Leu-CONH₂ | 139 |
| TCR134 | H₂N-AIB-Arg-Pro-Asp-Lys-Ala-Ala-Leu-Gly-Tyr-Asn-Orn-Arg-Pro-Ile-Pro-Asn-Ile-Leu-Val-Thr-Thr-Leu-CONH₂ | 140 |
| TCR135 | H₂N-AIB-Arg-Pro-Asp-Lys-Ala-Ala-Leu-Gly-Tyr-Asn-Orn-Arg-Pro-Ile-Pro-Asn-Ile-Leu-Ile-Thr-Thr-Leu-CONH₂ | 141 |
| TCR136 | H₂N-AIB-Arg-Pro-Asp-Lys-Ala-Ala-Leu-Gly-Tyr-Asn-Orn-Arg-Pro-Ile-Pro-Asn-Ile-Leu-Leu-Thr-Thr-Met-CONH₂ | 142 |
| TCR137 | H₂N-AIB-Arg-Pro-Asp-Lys-Ala-Ala-Leu-Gly-Tyr-Asn-Orn-Arg-Pro-Ile-Pro-Asn-Ile-Leu-Leu-Thr-Ser-Met-CONH₂ | 143 |
| TCR138 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ser-Ser-Met-CONH₂ | 144 |
| TCR139 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ser-Thr-Met-CONH₂ | 145 |
| TCR140 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ala-Thr-Met-CONH₂ | 146 |
| TCR141 | H₂N-AIB-Arg-Pro-Asp-Lys-Ala-Ala-Leu-Gly-Tyr-Asn-Orn-Arg-Pro-Leu-Pro-Gln-Val-Leu-Leu-Thr-Thr-Abu-CONH₂ | 147 |
| TCR142 | H₂N-AIB-Arg-Pro-Asp-Lys-Ala-Ala-Leu-Gly-Tyr-Asn-Orn-Arg-Pro-Leu-Pro-Gln-Val-Leu-Leu-Thr-Ser-Abu-CONH₂ | 148 |
| TCR143 | H₂N-AIB-Arg-Pro-Asp-Lys-Ala-Ala-Leu-Gly-Tyr-Asn-Orn-Arg-Pro-Leu-Pro-Gln-Val-Leu-Leu-Ser-Ser-Abu-CONH₂ | 149 |
| TCR144 | H₂N-AIB-Arg-Pro-Asp-Lys-Ala-Ala-Leu-Gly-Tyr-Asn-Orn-Arg-Pro-Leu-Pro-Gln-Val-Leu-Leu-Ser-Thr-Abu-CONH₂ | 150 |
| TCR145 | H₂N-AIB-Arg-Pro-Asp-Lys-Ala-Ala-Leu-Gly-Tyr-Asn-Orn-Arg-Pro-Leu-Pro-Gln-Val-Leu-Leu-Ala-Thr-Abu-CONH₂ | 151 |
| TCR146 | H₂N-AIB-Arg-Pro-Asp-Lys-Ala-Ala-Leu-Gly-Tyr-Asn-Orn-Arg-Pro-Ile-Pro-Asn-Ile-Leu-Leu-Thr-Thr-Abu-CONH₂ | 152 |
| TCR147 | H₂N-AIB-Arg-Pro-Asp-Lys-Ala-Ala-Leu-Gly-Tyr-Asn-Orn-Arg-Pro-Ile-Pro-Asn-Ile-Leu-Leu-Thr-Ser-Abu-CONH₂ | 153 |
| TCR148 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ser-Ser-Abu-CONH₂ | 154 |
| TCR149 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ser-Thr-Abu-CONH₂ | 155 |
| TCR150 | H₂N-AIB-Arg-Pro-Asp-Lys-Ala-Ala-Leu-Gly-Tyr-Asn-Orn-Arg-Pro-Ile-Pro-Asn-Ile-Leu-Leu-Ala-Thr-Abu-CONH₂ | 156 |
| TCR151 | H₂N-AIB-Arg-Pro-Glu-Orn-Ser-Ala-Leu-Gly-Tyr-Asn-Orn-Arg-Pro-Ile-Pro-Asn-Ile-Leu-Leu-Thr-Thr-Leu-CONH₂ | 157 |
| TCR152 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ala-Ala-Leu-CONH₂ | 158 |
| TCR153 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ala-Thr-Leu-CONH₂ | 159 |
| TCR155 | H₂N-AIB-Arg-Pro-Glu-Orn-Ser-Ala-Leu-Gly-Tyr-Asn-Orn-Arg-Pro-Ile-Pro-Asn-Ile-Leu-Leu-Thr-Thr-Val-CONH₂ | 160 |
| TCR156 | H₂N-AIB-Arg-Pro-Glu-Orn-Ser-Ala-Leu-Gly-Tyr-Asn-Orn-Arg-Pro-Ile-Pro-Asn-Ile-Leu-Leu-Thr-Ala-Val-CONH₂ | 161 |
| TCR157 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ala-Thr-Val-CONH₂ | 162 |
| TCR158 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ser-Thr-Val-CONH₂ | 163 |
| TCR159 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ser-Ser-Val-CONH₂ | 164 |
| TCR160 | H₂N-AIB-Arg-Pro-Glu-Orn-Ser-Ala-Leu-Gly-Tyr-Asn-Orn-Arg-Pro-Ile-Pro-Asn-Ile-Leu-Leu-Thr-Ser-Val-CONH₂ | 165 |
| TCR161 | H₂N-AIB-Arg-Pro-Glu-Orn-Ser-Ala-Leu-Gly-Tyr-Asn-Orn-Arg-Pro-Ile-Pro-Asn-Ile-Leu-Leu-Thr-Ser-AIB-CONH₂ | 166 |
| TCR162 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ser-Ser-AIB-CONH₂ | 167 |
| TCR163 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ser-Thr-AIB-CONH₂ | 168 |
| TCR164 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ala-Thr-AIB-CONH₂ | 169 |
| TCR165 | H₂N-AIB-Arg-Pro-Glu-Orn-Ser-Ala-Leu-Gly-Tyr-Asn-Orn-Arg-Pro-Ile-Pro-Asn-Ile-Leu-Leu-Thr-Thr-AIB-CONH₂ | 170 |
| TCR166 | H₂N-AIB-Arg-Pro-Glu-Orn-Ser-Ala-Leu-Gly-Tyr-Asn-Orn-Arg-Pro-Ile-Pro-Asn-Ile-Leu-Leu-Thr-Thr-Cys-CONH₂ | 171 |
| TCR167 | H₂N-AIB-Arg-Pro-Glu-Orn-Ser-Ala-Leu-Gly-Tyr-Asn-Orn-Arg-Pro-Ile-Pro-Asn-Ile-Leu-Leu-Thr-Ser-Cys-CONH₂ | 172 |
| TCR168 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ser-Ser-Cys-CONH₂ | 173 |
| TCR169 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ser-Thr-Cys-CONH₂ | 174 |
| TCR170 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ala-Thr-Cys-CONH₂ | 175 |
| TCR171 | H₂N-AIB-Arg-Pro-Glu-Orn-Ser-Ala-Leu-Gly-Tyr-Asn-Orn-Arg-Pro-Ile-Pro-Asn-Ile-Leu-Leu-Thr-Thr-Nle-CONH₂ | 176 |
| TCR172 | H₂N-AIB-Arg-Pro-Glu-Orn-Ser-Ala-Leu-Gly-Tyr-Asn-Orn-Arg-Pro-Ile-Pro-Asn-Ile-Leu-Leu-Thr-Ser-Nle-CONH₂ | 177 |
| TCR173 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ser-Ser-Nle-CONH₂ | 178 |
| TCR174 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ser-Thr-Nle-CONH₂ | 179 |
| TCR175 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ala-Thr-Nle-CONH₂ | 180 |
| TCR176 | H₂N-AIB-Arg-Pro-Glu-Orn-Ser-Ala-Leu-Gly-Tyr-Asn-Orn-Arg-Pro-Ile-Pro-Asn-Ile-Leu-Leu-Thr-Thr-Ala-CONH₂ | 181 |
| TCR177 | H₂N-AIB-Arg-Pro-Glu-Orn-Ser-Ala-Leu-Gly-Tyr-Asn-Orn-Arg-Pro-Ile-Pro-Asn-Ile-Leu-Leu-Thr-Ser-Ala-CONH₂ | 182 |
| TCR178 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ser-Ser-Ala-CONH₂ | 183 |
| TCR179 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ser-Thr-Ala-CONH₂ | 184 |
| TCR180 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ala-Thr-Ala-CONH₂ | 185 |
| TCR181 | H₂N-AIB-Arg-Pro-Glu-Orn-Ser-Ala-Leu-Gly-Tyr-Asn-Orn-Arg-Pro-Ile-Pro-Asn-Ile-Leu-Leu-Thr-Thr-Leu-CONH₂ | 186 |
| TCR182 | H₂N-AIB-Arg-Pro-Glu-Orn-Ser-Ala-Leu-Gly-Tyr-Asn-Orn-Arg-Pro-Ile-Pro-Asn-Ile-Val-Val-Thr-Thr-Leu-CONH₂ | 187 |
| TCR183 | H₂N-AIB-Arg-Pro-Glu-Orn-Ser-Ala-Leu-Gly-Tyr-Asn-Orn-Arg-Pro-Ile-Pro-Asn-Ile-Val-Leu-Thr-Thr-Leu-CONH₂ | 188 |
| TCR184 | H₂N-AIB-Arg-Pro-Glu-Orn-Ser-Ala-Leu-Gly-Tyr-Asn-Orn-Arg-Pro-Ile-Pro-Asn-Ile-Leu-Val-Thr-Thr-Leu-CONH₂ | 189 |
| TCR185 | H₂N-AIB-Arg-Pro-Glu-Orn-Ser-Ala-Leu-Gly-Tyr-Asn-Orn-Arg-Pro-Ile-Pro-Asn-Ile-Leu-Ile-Thr-Thr-Leu-CONH₂ | 190 |
| TCR186 | H₂N-AIB-Arg-Pro-Glu-Orn-Ser-Ala-Leu-Gly-Tyr-Asn-Orn-Arg-Pro-Ile-Pro-Asn-Ile-Leu-Leu-Thr-Thr-Met-CONH₂ | 191 |
| TCR187 | H₂N-AIB-Arg-Pro-Glu-Orn-Ser-Ala-Leu-Gly-Tyr-Asn-Orn-Arg-Pro-Ile-Pro-Asn-Ile-Leu-Leu-Thr-Ser-Met-CONH₂ | 192 |
| TCR188 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ser-Ser-Met-CONH₂ | 193 |
| TCR189 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ser-Thr-Met-CONH₂ | 194 |
| TCR190 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ala-Thr-Met-CONH₂ | 195 |
| TCR191 | H₂N-AIB-Arg-Pro-Glu-Orn-Ser-Ala-Leu-Gly-Tyr-Asn-Orn-Arg-Pro-Leu-Pro-Gln-Val-Leu-Leu-Thr-Thr-Abu-CONH₂ | 196 |
| TCR192 | H₂N-AIB-Arg-Pro-Glu-Orn-Ser-Ala-Leu-Gly-Tyr-Asn-Orn-Arg-Pro-Leu-Pro-Gln-Val-Leu-Leu-Thr-Ser-Abu-CONH₂ | 197 |
| TCR193 | H₂N-AIB-Arg-Pro-Glu-Orn-Ser-Ala-Leu-Gly-Tyr-Asn-Orn-Arg-Pro-Leu-Pro-Gln-Val-Leu-Leu-Ser-Ser-Abu-CONH₂ | 198 |
| TCR194 | H₂N-AIB-Arg-Pro-Glu-Orn-Ser-Ala-Leu-Gly-Tyr-Asn-Orn-Arg-Pro-Leu-Pro-Gln-Val-Leu-Leu-Ser-Thr-Abu-CONH₂ | 199 |
| TCR195 | H₂N-AIB-Arg-Pro-Glu-Orn-Ser-Ala-Leu-Gly-Tyr-Asn-Orn-Arg-Pro-Leu-Pro-Gln-Val-Leu-Leu-Ala-Thr-Abu-CONH₂ | 200 |
| TCR196 | H₂N-AIB-Arg-Pro-Glu-Orn-Ser-Ala-Leu-Gly-Tyr-Asn-Orn-Arg-Pro-Ile-Pro-Asn-Ile-Leu-Leu-Thr-Thr-Abu-CONH₂ | 201 |
| TCR197 | H₂N-AIB-Arg-Pro-Glu-Orn-Ser-Ala-Leu-Gly-Tyr-Asn-Orn-Arg-Pro-Ile-Pro-Asn-Ile-Leu-Leu-Thr-Ser-Abu-CONH₂ | 202 |
| TCR198 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ser-Ser-Abu-CONH₂ | 203 |
| TCR199 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ser-Thr-Abu-CONH₂ | 204 |
| TCR200 | H₂N-AIB-Arg-Pro-Glu-Orn-Ser-Ala-Leu-Gly-Tyr-Asn-Orn-Arg-Pro-Ile-Pro-Asn-Ile-Leu-Leu-Ala-Thr-Abu-CONH₂ | 205 |

The polypeptide compounds in the present disclosure can be synthesized and confirmed by the following processes.

### 1. Synthesis of Polypeptide Compounds

Abbreviations used in the present disclosure have the following meanings:

| | | | |
|---|---|---|---|
| Fmoc: | Fluorenylmethoxycarbonyl | DIC: | N,N-Diisopropylcarbodiimide |
| Boc: | tert-Butoxycarbonyl | NMP: | N-Methylpyrrolidone |
| DIEA: | Diisopropylethylamine | TFA: | Trifluoroacetic acid |
| DCM: | Dichloromethane | TIS: | Triisopropylsilane |
| DCE: | Dichloroethane | DTT: | Dithiothreitol |
| DMF: | N,N-Dimethylformamide | Mesph: | Thioanisole |
| Oxyma: | Ethyl Cyanoglyoxylate-2-Oxime | Meoph: | Anisole |

The sources of reagents used in this Example are as follows:
Fmoc-Rink Amide-AM resin was purchased from Xi'an Sunresin New Materials Co., Ltd.; Fmoc-AA-OH amino acids were purchased from Chengdu zyiochemical Technology Co., Ltd.; DMF, DCM, and TFA were purchased from Concord Technology (Tianjin) Co., Ltd.; DIEA, DIC, Oxyma, and DTT were purchased from Suzhou Highfine Biotech Co., Ltd.; Tis was purchased from Shanghai Shaoyuan Co., Ltd.; unless otherwise specified, other reagents were purchased from Sinopharm or Aladdin.

### Synthesis of Polypeptides

(1) Using the solid-phase synthesis method, the carrier resin was selected according to the structure of the peptide chain to be synthesized. When the C-terminus is an amide, Fmoc-Rink Amide-AM resin was selected;
(2) Coupling of the 1^{st} amino acid on the resin (Fmoc-Rink Amide-AM resin coupling method is consistent with amino acid coupling method): After the resin was swollen in DCM, 1.5 eq of Fmoc-AA-OH and 7.5 eq of DIEA were added thereto, using DCM as the solvent, which were reacted at room temperature for 30-60 min. The reaction solution was drained and the resin was washed. The remaining sites on the carrier were capped with a reaction solution of MeOH:DIEA:DCM = 2:1:17 (volume ratio), and they were reacted at room temperature for 10-30 min, and washed;
(3) Amino acid coupling: 10 times equivalent (v/m) of 20% piperidine/DMF solution was added to the aforementioned swollen resin, they were reacted at room temperature for 5-10 min, then the reaction solution was drained, and the resin was washed three times with 10 times equivalent (v/m) of DMF and DCM, respectively. After draining, a resin with exposed amino groups was obtained; the amino acid condensation reaction solution (consisting of 3eq of Fmoc-AA-OH, 3eq of Oxyma, and 3eq of DIC) was used for coupling at room temperature for 30-120 min, with 10 times equivalent (v/m) of DMF being used as solvent; after the reaction was completed, 10 times equivalent (v/m) of DMF was added to wash the resin. Ninhydrin test was conducted and showed negative;
(4) Operation of (3) was repeated, and the Fmoc-AA-OH of subsequent sites were sequentially coupled to the target peptide resin according to the amino acid sequence. The Fmoc protecting group of the last amino acid at the end of the resin was removed. After washing and shrinking, the peptide resin was dried to constant weight;
(5) A cleavage reagent (TFA:TIS:DTT:Mesph:Meoph:H₂O = 80:1:5:5:5:4, by volume) was used to cleave the peptide chain from the resin. After the resin was filtered, the resin was washed with TFA (1.2 eq by v/m), the filtrates were combined, and part of the TFA was removed by rotary evaporation;
(6) Methyl tert-butyl ether was added to the filtrate obtained in step (5) at a volume ratio of filtrate:methyl tert-butyl ether = 1:5, and centrifuged. The obtained filter cake was washed with methyl tert-butyl ether, centrifuged three times, and vacuum dried to obtain the target linear polypeptide;
(7) The crude polypeptide was dissolved (or the cyclized reaction solution was taken). After being filtrated with 0.22 µm microporous filter, it was prepared, separated, and purified using preparative liquid chromatograph via gradient elution. Mobile phase A was 0.1% TFA and water, mobile phase B was acetonitrile, and the flow rate was 1.0 mL/min; the target polypeptide compound was separated and prepared, then lyophilized to obtain the pure product.

### 2. Confirmation of Polypeptide Compounds

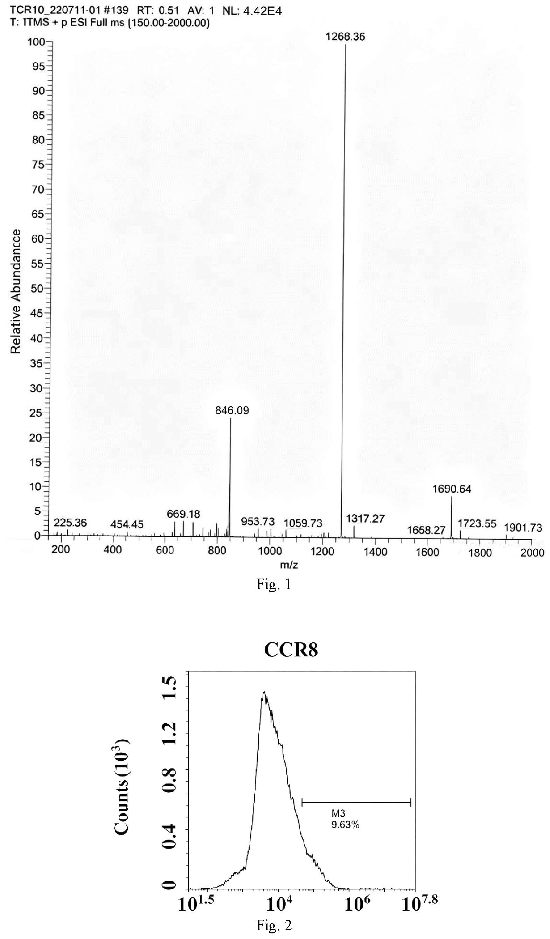

The polypeptide compounds synthesized in item 1 above were identified by HPLC and structurally analyzed by Thermo Fisher LTQ XL linear ion trap mass spectrometer. Chromatographic mobile phases were acetonitrile and water. Mobile phase A was 0.1% TFA and water, mobile phase B was acetonitrile, and the elution gradient was adjusted according to different target products. The measured retention time was consistent with that of the main peak of the crude product, and the purity of the polypeptides prepared in this Example was greater than 90%. Taking TCR10 as an example, the analysis result by LTQ XL linear ion trap mass spectrometer is shown in Fig. 1. The specific information of the polypeptide compound TCR10 synthesized in this Example is shown in Table 1 below.

**Table 1 Final Product Information of Polypeptide Compound TCR10**

| No. | Polypeptide Compound Code | Lyophilized Product (mg) | Yield | HPLC Purity | MS Value |
|---|---|---|---|---|---|
| 1 | TCR10 | 271.5 | 53.55% | 95.62% | 2535 |

As identified by HPLC and structurally analyzed by LTQ XL linear ion trap mass spectrometer, the HPLC purities of the prepared TCR01-TCR09 and TCR11-TCR200 were all greater than 90%.

Next, the activities of the prepared exemplary CCR8 antagonistic polypeptide compounds were verified through the following Experimental Examples.

Atopic dermatitis is a chronic relapsing skin disease with a steadily increasing prevalence in children, reaching 10%-20%. Skin-infiltrating T cells, dendritic cells, and mast cells are considered to play crucial roles in its pathogenesis. It is reported that compared with psoriasis, cutaneous lupus erythematosus or normal skin, the expression of the CC chemokine CCL1 (I-309) is significantly and selectively upregulated in atopic dermatitis. Serum levels of CCL1 in patients with atopic dermatitis are significantly higher than those in healthy individuals. DCs, mast cells, and dermal endothelial cells are abundant sources of CCL1 during atopic dermatitis and allergen challenge. Patients with atopic dermatitis exhibit skin barrier dysfunction, frequent allergic reactions to allergens, defects in antimicrobial immune defense, and genetic predisposition. Clinical observations of AD combined with the results of recent scientific research suggest an amplification cycle in atopic dermatitis. These amplification processes sustain the inflammatory response within the skin and lead to the development of an AD phenotype. Studies demonstrate that the CCL1-CCR8 axis links adaptive and innate immune functions, which play roles in the initiation and amplification of atopic dermatitis ^{[1-2]}.

In the following Experimental Examples 1-2, SPR was used to determine the binding ability of polypeptide compounds to the CCR8 receptor, and the transwell chemotaxis assay was used to evaluate whether polypeptide compounds could inhibit cell migration by blocking the CCL1-CCR8 axis. In the following Experimental Examples 3-5, female Balb/c mice weighing 18-22 g were used; and a solution of 50mM MC903 (calcipotriol) in 75% ethanol was used as a modeling agent. MC903, i.e., calcipotriol, is a low-calcium analog of vitamin D3 widely used for treating psoriasis. Topical application of MC903 can stimulate epithelial keratinocytes in mice to highly express TSLP, promote differentiation of CD4+ T cells into Th2 cells, and activate and release inflammatory cytokines, causing mice to develop an atopic dermatitis phenotype.

### Experimental Example 1: In Vitro SPR Affinity Assay of Polypeptide Compounds with CCR8 Protein

Human CCR8 full-length protein (VLP) chips were prepared by amino coupling method. CCR8 full-length protein (VLP) was immobilized on two channels of a CM5 chip. This Experimental Example tested the strength of affinity of polypeptide compounds with CCR8 protein, to confirm their binding ability to the CCR8 receptor.

### (1) Experimental Protocol

Vehicle for test compounds (TCR10 and TCR09): PBS solution (containing 0.05% Tween 20).

The polypeptide compounds were formulated into 1 mM solutions as drug stock solutions, and then serially diluted with the PBS solution into working solutions with concentrations of 2.56×10⁻² nM, 0.128 nM, 0.64 nM, 3.2 nM, 16 nM, 80 nM, 400 nM, 2 µM, and 10 µM, using the PBS solution as a blank control at the same time. The working solutions of the above concentrations were captured on the Fc3 and Fc4 channels of the CCR8(VLP)-CM5 chip using the PBS solution. The polypeptide compound samples to be tested at various concentrations and the blank control flowed through the test channels (Fc3 and Fc4) respectively, at a flow rate of 30 µL/min, with an association time of 120 seconds and a dissociation time of 120 seconds. After each test, the chip was regenerated with Gly-HCl buffer of pH 1.5.

### (2) Experimental Results

The experimental results are shown in Table 2. The affinities of TCR10 and TCR09 binding to human CCR8 protein were 17.17 nM and 228.8 nM, respectively.

**Table 2 Affinity of Polypeptide Compounds with Human CCR8 Protein**

| Compound | Kₐ (1/Ms) | K_{d} (1/s) | K_{D} (M) |
|---|---|---|---|
| TCR10 | 1.550E+5 | 2.662E-3 | 1.717E-8 |
| TCR09 | 3.326E+3 | 7.608E-4 | 2.288E-7 |

### Experimental Example 2: Transwell Chemotaxis Assay for Evaluating the Effect of Polypeptide Compounds on Cell Migration

PBMC-derived interstitial dendritic cells were induced to differentiate, and the migration number of interstitial dendritic cells in the presence or absence of polypeptide compounds was counted using the Transwell chemotaxis assay. This Experimental Example was used to evaluate whether polypeptide compounds block the migration of interstitial dendritic cell through the CCL1-CCR8 axis.

### (1) Experimental Protocol

Vehicle for test compound (TCR10): PBS solution + RPMI 1640.

Antibody control: Anti-CCR8 antibody (purchased from Genomeditech, GM-30913AB); Vehicle: RPMI 1640.

CD14⁺ monocytes were sorted from human PBMCs using magnetic beads and cultured in the presence of GM-CSF and IL-4 for 6 days to obtain interstitial dendritic cells. Expressions of CD209, CD1α, and CCR8 were detected by flow cytometry. The differentiated interstitial dendritic cells were collected and added to the upper chamber of the transwell. Samples were distributed to the upper and lower chambers of each chamber according to Table 3,wherein in the blank control group (abbreviated as "blank group"), only cells were added; in the positive control group, CCL1 and CXCL12 were added; in the test compound group, test compound plus CCL1 and CXCL12 were added; in the antibody control group, anti-CCR8 antibody plus CCL1 and CXCL12 were added; each group was tested in duplicate. Migration was performed in a 37°C, 5% CO₂ incubator for 5 hours. After fixation with formaldehyde and staining with crystal violet, cells that did not migrate in the chamber were gently wiped off with a cotton swab. Cells that migrated through the chamber were counted using a microscope.

**Table 3 Group Setting and Dosing**

| Transwell chamber | Sample | Blank group | Positive control group | Test compound group | Antibody control group |
|---|---|---|---|---|---|
| Upper chamber | Test compound (final concentration 0.5 µg/mL) | - | - | + | - |
| | Anti-CCR8 antibody (final concentration 0.5 µg/mL) | - | - | - | + |
| Lower chamber | CCL1 (final concentration 500 ng/mL) | - | + | + | + |
| | CXCL12 (final concentration 1 ng/mL) | - | + | + | + |

### (2) Experimental Indicator: Migration number of interstitial dendritic cells.

### (3) Experimental Results:

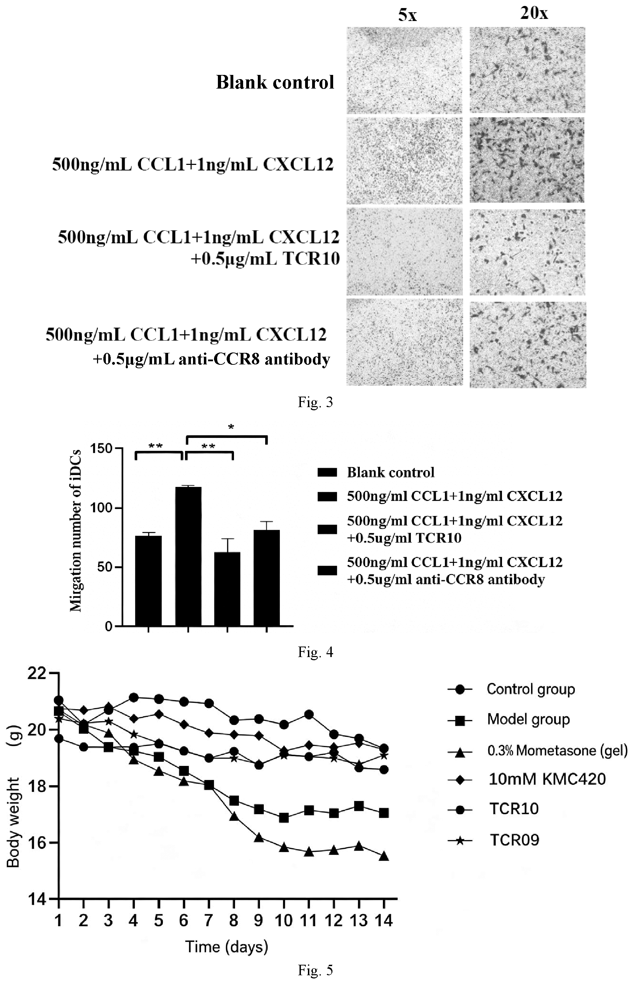

Flow cytometry results showed that CCR8 was expressed on the surface of interstitial dendritic cells (see Fig. 2). As shown in Figs. 3 and 4, compared with the blank group, interstitial dendritic cells in the positive control group responded to chemotaxis and migrated significantly. After adding TCR10 or anti-CCR8 antibody, the migration amount of interstitial dendritic cells (iDCs) decreased. In particular, the decrease in cell migration amount was more significant after adding TCR10, indicating that TCR10 can block the CCL1-CCR8 axis, thereby affecting the migration of interstitial dendritic cells.

### Experimental Example 3: Drug efficacy Screening (Preliminary Screening) based on Calcipotriol-Induced Mouse Atopic Dermatitis Model

After shaving the dorsal skin of mice, MC903 was applied once a day (50 µL was applied on dorsal skin with the area of 2cm*2cm, and 20 µL was applied on ear) to induce atopic dermatitis in mice. This Experimental Example was used to test the efficacy of the CCR8 antagonistic polypeptides in the present disclosure for preventing atopic dermatitis.

### (1) Experimental Protocol

Vehicle for test compounds (TCR10 and TCR09): Normal saline.

Positive control drugs: 0.3 wt% mometasone gel (glucocorticoid) and KMC420 (JAK inhibitor, 10 mM; vehicle: normal saline).

Mice in the normal control group were only treated with 75% ethanol solution for 14 days without MC903-induced modeling. Mice in the model group were only applied with MC903 every day for 14 consecutive days. The dosage is shown in the table below. During the administration, mice in the positive control drug group and test compound (TCR9 and TCR10) groups were first applied with the positive control drug (application amounts for mometasone gel and KMC420 both were 50 µL on back, and 20 µL on ear (10 µL on each of the inner and outer sides)) or test compound (TCR10 and TCR09, both applied at a concentration of 20 mg/mL with 50 µL on back, and both applied at a concentration of 50 mg/mL with 10 µL on each of the inner and outer sides of the ear) every day. After drying thoroughly, the modeling agent was applied (50 µL was applied on dorsal skin, and 20 µL was applied on ear (10 µL on each of the inner and outer sides) each time), once a day for 14 consecutive days.

**Table 4 Compound Test Protocol for Drug efficacy Screening Test**

| Group | Number of animals (n) | Dosing mode | Dosage | Dosing frequency | Period |
|---|---|---|---|---|---|
| Normal control group | 2 | Topical application of 75% EtOH | back: 50 µL, ear: 20 µL | qd | 14 days |
| Model group | 2 | Topical application of MC903 | back: 50 µL, ear: 20 µL | qd | 14 days |
| Mometasone gel group | 2 | Topical application (drug→MC903) | back: 50 µL, ear: 20 µL | qd | 14 days |
| KMC420 group | 3 | Topical application (drug→MC903) | back: 50 µL, ear: 20 µL | qd | 14 days |
| TCR10 group | 2 | Topical application (peptide→MC903) | back: 50 µL, ear: 20 µL | qd | 14 days |
| TCR09 group | 2 | Topical application (peptide→MC903) | back: 50 µL, ear: 20 µL | qd | 14 days |

Before modeling with MC903, the body weight, remaining food amount, and ear thickness of mice in each group were measured, and then, before daily administration, the body weight, remaining food amount, and ear thickness of mice in each group were measured. Photographs were taken and skin lesions were scored regularly on the ear and dorsal skin. After the last administration, ear and dorsal skin, liver, kidney, and spleen of mice were collected and fixed in formalin solution for pathological testing.

(2) Experimental Indicators: Mouse weight, Ear swelling degree, Skin lesion score, Pathological analysis.

**Table 5 Mouse Skin Lesion Scoring Criteria**

| Skin lesion evaluation | | Grade | | | |
|---|---|---|---|---|---|
| Symptoms | | 0 | 1 | 2 | 3 |
| 1) | Erythema/Color Darkening | No redness or swelling | Slight redness | Patchy redness, color darkening | Extensive redness, accompanied by uneven distribution of deep red with bleeding |
| 2) | Edema/Papules (on the basis of erythema) | No edema | Slight edema, not obvious to touch | Obvious protrusion resulting in thickening | Multiple papules, hardening, animal squeaking upon palpation |
| 3) | Exudation/Scabbing | No exudation or scabbing | Hair clumping, slight yellowing | Obvious clumping, yellow | Large cracks, deepening yellow, with exudate |
| | Peeling/Fissure | No fissuring or peeling | Scabs slightly peeling, no bleeding | Large area fissuring, peeling with bleeding | Almost complete peeling |
| 4) | Lichenification/Itching & Pain (after scabbing and shedding) | No symptom | Fine and dense, occasional itching | Obvious epidermis thickening, with multiple fine and shallow lines / obvious itching | Obvious epidermis thickening, bark-like lines / itching |
| | Dryness | No dryness | Dry without scaling | Small amount of scales | Large area of scales |

### (3) Experimental Results

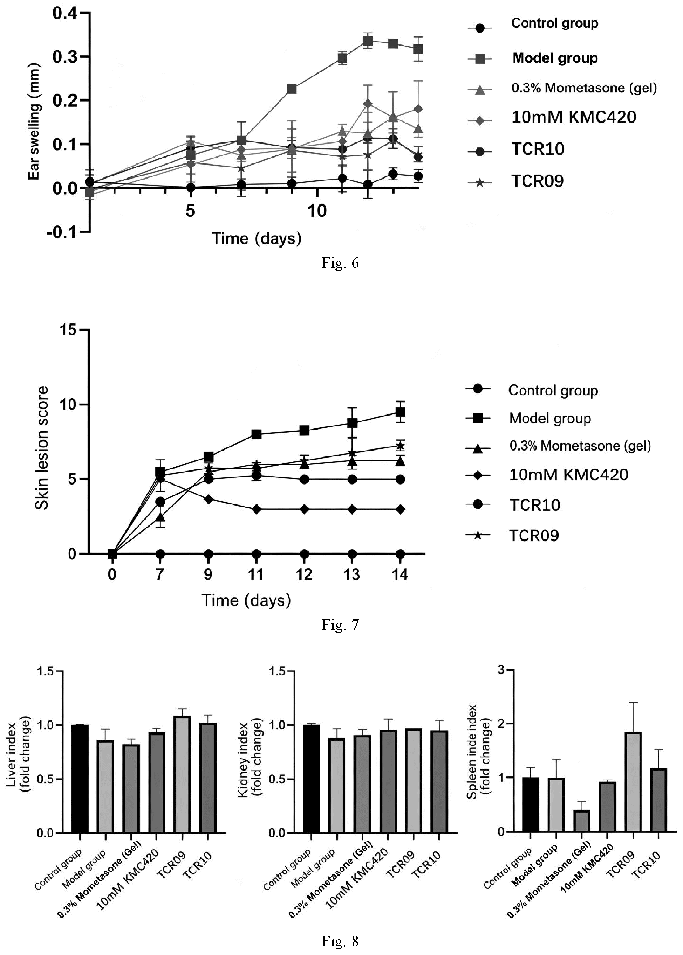

The Experimental results are shown in Fig. 5 to Fig. 10, wherein compared with the model group, the administration groups of the CCR8 antagonistic polypeptide compounds of the present disclosure significantly improved the weight loss of mice, which was substantially equivalent to the normal control group (see Fig. 5); compared with the model group, the degree of mouse ear swelling in the administration groups of the CCR8 antagonistic polypeptides of the present disclosure was significantly alleviated, substantially reaching the level of normal mice (see Fig. 6), the dorsal skin lesion scores were close to normal mice (see Fig. 7), and the liver, kidney, and spleen indices were comparable or substantially close to normal mice (see Fig. 8).

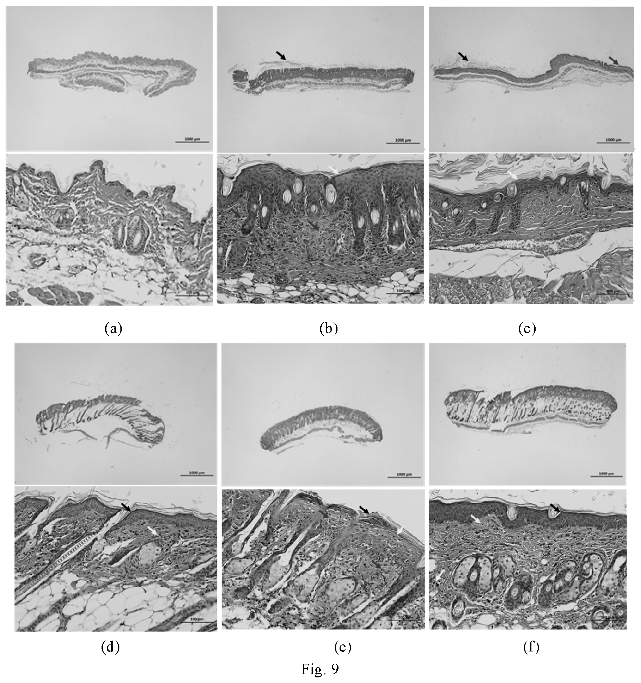

As shown in Fig. 9, it was found by the analysis on the pathological section of mouse dorsal skin that the epidermis of skin tissue of the mice in the normal control group was intact with clear structure and visible keratinized layer, collagen fibers in the dermis were arranged neatly, skin appendages such as hair follicles were scattered; subcutaneous tissue was located beneath the dermis, consisting of loose connective tissue, adipose tissue and muscle layer, with no obvious abnormalities observed (Fig. 9(a)). For mice in the model group, there was local stratum corneum hyperplasia (black arrow) and moderate thickening of epidermis (yellow arrow), and scattered lymphocyte and neutrophil infiltration could be observed in the dermis (red arrow) (Fig. 9(b)). For mice in the 0.3% mometasone gel administration group, there was local stratum corneum hyperplasia (black arrow), and widespread epidermis thickening (yellow arrow) and local epidermis (red arrow) could be observed (Fig. 9(c)). For mice in the KMC420 administration group, widespread epidermis thickening (black arrow) was observed in the tissue, and a small amount of lymphocyte infiltration could be observed in the dermis (yellow arrow) (Fig. 9(d)). For mice in the TCR09 administration group, there was rare parakeratosis in the stratum corneum (black arrow) and moderate thickening of the epidermis (yellow arrow), and scattered lymphocyte and neutrophil infiltration could be observed in the epidermis and dermis (red arrow) (Fig. 9(e)). For mice in the TCR10 administration group, widespread epidermis thickening (black arrow) could be observed in the tissue, and scattered lymphocyte and neutrophil infiltration could be observed in the dermis (yellow arrow) (Fig. 9(f)).

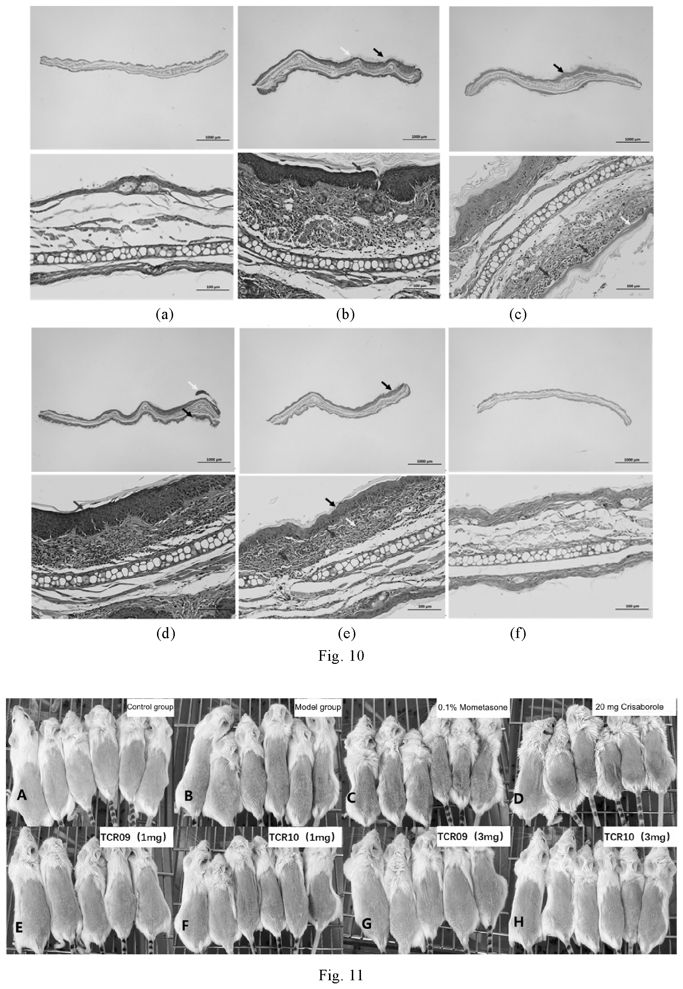

As shown in Fig. 10, it was found by the analysis on the pathological section of mouse ear that the epidermis of ear tissue in the mice of the normal control group was intact with clear structure, keratinized layer could be observed, collagen fibers in the dermis were arranged neatly, skin appendages such as hair follicles and sebaceous glands were scattered, subcutaneous tissue was located beneath the dermis, consisting of loose connective tissue, adipose tissue and muscle layer, with no obvious abnormalities observed (Fig. 10(a)). For mice in the model group, stratum corneum hyperplasia was frequently observed (black arrow), local parakeratosis (yellow arrow) and moderate thickening of epidermis (red arrow) could be observed, substantial lymphocyte and neutrophil infiltration could be observed in the dermis (blue arrow), small amount of connective tissue hyperplasia (green arrow) could be observed locally, accompanied by a small amount of neovascularization (brown arrow) (Fig. 10(b)). For mice in the 0.3% mometasone gel administration group, widespread epidermis thickening (black arrow), substantial epidermal cell necrosis, and karyolysis (yellow arrow), substantial lymphocyte and neutrophil infiltration in the surrounding dermis (red arrow), and a small amount of venous congestion and dilation (blue arrow) were observed (Fig. 10(c)). For mice in the KMC420 administration group, multiple areas of stratum corneum thickening (black arrow), local large abscesses (yellow arrow), widespread epidermis thickening (red arrow) in the tissue, moderate lymphocyte and neutrophil infiltration in the dermis (blue arrow) could be observed, accompanied by mild connective tissue hyperplasia (green arrow) (Fig. 10(d)). For mice in the TCR09 administration group, widespread mild thickening of the epidermis with more pronounced local thickening (black arrow) in the tissue, and mild connective tissue hyperplasia locally in the epidermis (yellow arrow) could be observed, accompanied by lymphocyte and neutrophil infiltration (red arrow) (Fig. 10(e)). For mice in the TCR10 administration group, the epidermis of ear tissue was intact with clear structure, keratinized layer could be observed, collagen fibers in the dermis were arranged neatly, skin appendages such as hair follicles and sebaceous glands were scattered, subcutaneous tissue was located beneath the dermis, consisting of loose connective tissue, adipose tissue and muscle layer, with no obvious abnormalities (Fig. 10(f)).

### Experimental Example 4: Dose-Effect Test

After shaving the dorsal skin of mice, MC903 was applied once a day (50 µL was applied on dorsal skin with the area of 2cm*2cm, and 10 µL was applied on each of the inner and outer sides of ear) to induce atopic dermatitis in mice. For mice in the administration groups (including positive control drug group and test compound group), drugs were pre-applied before each application of MC903 to test the preventive effect of drugs on atopic dermatitis.

### (1) Experimental Protocol

Test compound solution: DMSO was used to dissolve the drug to a suitable concentration as a stock solution (stock solution for back: 200 mg/mL; stock solution for ear: 500 mg/mL). Then working solutions were prepared according to the following formula (by volume) and stored at 4°C: (1) 10% DMSO stock solution + 40% PEG300 + 5% Tween 80 + 45% NS (normal saline), resulting in final working solution having drug concentrations of: 20 mg/mL of working solution for back; 50 mg/mL of working solution for ear; (2) 30% DMSO stock solution + 40% PEG300 + 5% Tween 80 + 25% NS (normal saline), resulting in final working solution having drug concentrations of: 60 mg/mL of working solution for back; 150 mg/mL of working solution for ear.

Positive control drugs: 0.1 wt% mometasone furoate and 2 wt% crisaborole ointment.

Mice in the normal control group were only treated with 75% ethanol solution for 14 days without MC903-induced modeling. Mice in the model group were only applied with MC903 every day for 14 consecutive days. The dosage is shown in the table below. During the administration, mice in the positive control drug group and test compound (TCR9 and TCR10) groups were first applied with the positive control drug (application amount for 0.1% mometasone furoate was 50 µL on back, and 20 µL on ear (10 µL on each of the inner and outer sides); application amount for 2% crisaborole ointment was 20 mg on back, and 20 mg on ear (10 mg on each of the inner and outer sides)) or test polypeptide compound (TCR09 (1mg) and TCR10 (1mg) were applied at a concentration of 20 mg/mL with 50 µL on back, and applied at a concentration of 50 mg/mL with 10 µL on each of the inner and outer sides of the ear; TCR09 (3mg) and TCR10 (3mg) were applied at a concentration of 60 mg/mL with 50 µL on back, and at a concentration of 150 mg/mL with 10 µL on each of the inner and outer sides of the ear) every day. After drying thoroughly, the modeling agent was applied (50 µL was applied on dorsal skin, and 20 µL was applied on ear (10 µL on each of the inner and outer sides) each time) once a day for 14 consecutive days.

**Table 6 Compound Test Protocol for Dose-Effect Test**

| Group | | Number. of animals (n) | Dosing mode | Dosing frequency | Period | Dosage |
|---|---|---|---|---|---|---|
| Normal control group | | 6 | Topical application of 75% EtOH | qd | 14 days | back: 50 µL, each of the inner and outer sides of ear: 10 µL |
| Model group (MC903) | | 6 | Topical application of MC903 | qd | 14 days | back: 50 µL, each of the inner and outer sides of ear: 10 µL |
| Positive control group | 0.1% mometasone furoate | 6 | Topical application (drug→MC903) | qd | 14 days | back: 50 µL, each of the inner and outer sides of ear: 10 µL |
| | 2% crisaborole ointment | 6 | Topical application (drug→MC903) | Bid | 14 days | back: 20 mg, each of the inner and outer sides of ear: 10 mg |
| TCR09 peptide group | 1 mg TCR09 | 5 | Topical application (peptide→MC903) | qd | 14 days | back: 50 µL, each of the inner and outer sides of ear: 10 µL |
| | 3 mg TCR09 | 5 | Topical application (peptide→MC903) | qd | 14 days | back: 50 µL, each of the inner and outer sides of ear: 10 µL |
| TCR10 peptide group | 1 mg TCR10 | 6 | Topical application (peptide→MC903) | qd | 14 days | back: 50 µL, each of the inner and outer sides of ear: 10 µL |
| | 3 mg TCR10 | 6 | Topical application (peptide→MC903) | qd | 14 days | back: 50 µL, each of the inner and outer sides of ear: 10 µL |

After the last administration, the dorsal skin of mice in each group was photographed.

### (2) Experimental Indicator

Observation and monitoring of mouse skin lesions.

### (3) Experimental Results

The experimental results of the dose-effect test are shown in Fig. 11.

As shown in Fig. 11, compared with the model group, both of the 3mg TCR09 group and 3mg TCR10 group could significantly improve the dorsal skin lesions of mice, wherein the 3mg TCR10 group had the best improvement effect.

### Experimental Example 5: Efficacy Evaluation of Gel Formulation Treatment Model

After shaving the dorsal skin of mice, MC903 was applied once a day (50 µL was applied on dorsal skin with the area of 2cm*2cm). Successful modeling was confirmed by the degree of dorsal skin lesions in mice, obtaining a mouse atopic dermatitis model.

### (1) Experimental Protocol

Drug Stock Solution: The required amount of drug (TCR10) was weighed and fully dissolved in DMSO to obtain a stock solution, wherein the stock solution for back had a concentration of 200 mg/mL; and the stock solution for ear had a concentration of 500 mg/mL.

Gel Matrix: HPMC (hydroxypropyl methylcellulose) in an amount of 1.5 wt% of the final gel matrix amount was weighed, water was added thereto and stirred, swollen fully overnight; glycerol at a ratio of 20% and Tween 80 at a ratio of 0.5% relative to the total weight of the gel matrix, as well as an appropriate amount of triethanolamine (adjusting the pH of the system to 7-8) were added thereto and stirred uniformly to obtain it.

Gel Formulation: The drug stock solution was mixed with an appropriate amount of gel matrix, water was added to the full amount, and the gel was stirred to be uniform to obtain 2 wt% and 5 wt% TCR10 gel formulations.

Positive Control Drugs: 0.1 wt% mometasone furoate gel and 2 wt% crisaborole ointment.

Mice in the normal control group (Normal group) were only treated with 75% ethanol solution for 14 days, once a day, without MC903-induced modeling. Mice in the model group were applied with MC903 once a day for 14 consecutive days. The dosage is shown in the table below. After continuous application of MC903 for modeling for 7 days, mice in the following groups were administered every day after MC903 application: for the gel matrix control group, 50 mg of gel matrix was applied to dorsal skin, and 25 mg of gel matrix was applied to ear skin (12.5 mg on each of the inner and outer sides), twice a day for 7 consecutive days; for the mometasone furoate gel group, 50 µL of 0.1% mometasone furoate gel was applied to dorsal skin, and 17 µL of 0.1% mometasone furoate gel was applied to ear skin (8.5 µL on each of the inner and outer sides) once a day for 7 consecutive days; for the crisaborole ointment group, 20 mg of 2% crisaborole ointment was applied to dorsal skin, and 7 mg of 2% crisaborole ointment was applied to ear skin (3.5 mg on each of the inner and outer sides) twice a day for 7 consecutive days; for the TCR10 gel group, 50 mg of 2% and 5% TCR10 gels were each applied to dorsal skin, and 25 mg were each applied to ear skin (12.5 mg on each of the inner and outer sides) twice a day for 7 consecutive days.

**Table 7 Dosing Protocol for Gel Formulation Treatment Model**

| Group | Concentration | Dosage | Number (N) | MC903 (times/ day) | Modeling period (days) | Dosing (times/ day) | Treatment period (days) |
|---|---|---|---|---|---|---|---|
| Normal control group | / | / | 8 | / | / | / | / |
| Model group | / | / | 8 | 1 | 14 | / | / |
| Gel matrix control group | / | back: 50 mg | 8 | 1 | 14 | 2 | 7 |
| | | ear: 25 mg | | | | | |
| Mometasone furoate group | 0.1% | back: 50 µL | 8 | 1 | 14 | 1 | 7 |
| | | ear: 17 µL | | | | | |
| Crisaborole group | 2% | back: 20mg | 8 | 1 | 14 | 2 | 7 |
| | | ear: 7mg | | | | | |
| TCR10 gel group | 2% | back: 50mg | 8 | 1 | 14 | 2 | 7 |
| | | ear: 25mg | | | | | |
| | 5% | back: 50mg | 8 | 1 | 14 | 2 | 7 |
| | | ear: 25mg | | | | | |

Before modeling with MC903, the body weight, remaining food amount, and ear thickness of mice in each group were measured. Subsequently, before daily administration, the body weight, remaining food amount, and ear thickness of mice in each group were measured. Photographs were taken and skin lesions were scored regularly on the dorsal skin. After the last administration, mouse dorsal skin was collected to measure cytokine content and epidermal thickness, and count skin mast cell, and part of the skin samples were fixed in formalin solution for pathological testing.

### (2) Experimental Indicators

Mouse body weight, mouse skin lesion scoring (scoring criteria were the same as Experimental Example 1), mouse ear swelling degree, cytokine content per mg skin of mouse (IL-4), mouse HE scoring, mouse epidermal thickness, mouse skin mast cell count and histopathological section.

**Table 8 HE Scoring Criteria**

| Rash | | Dryness | | Edema | |
|---|---|---|---|---|---|
| Scoring criteria | Score | Scoring Criteria | Score | Scoring Criteria | Score |
| Normal | 0 | Normal | 0 | Normal | 0 |
| Mild | 1 | Mild | 1 | Mild | 1 |
| Mild-Moderate | 2 | Mild-Moderate | 2 | Mild-Moderate | 2 |
| Moderate-Severe | 3 | Moderate-Severe | 3 | Moderate-Severe | 3 |
| Severe | 4 | Severe | 4 | Severe | 4 |

### (3) Experimental Results

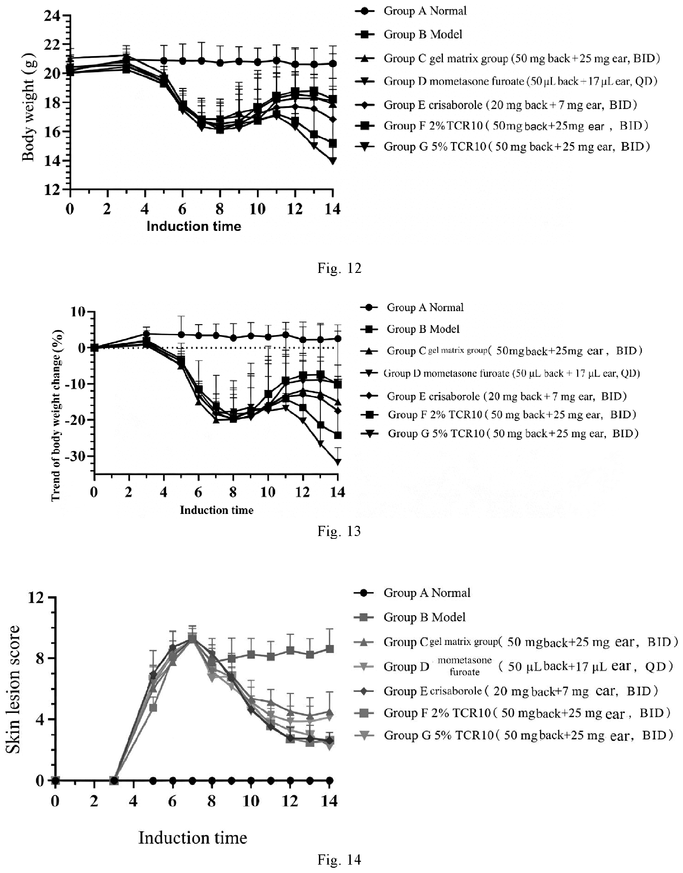

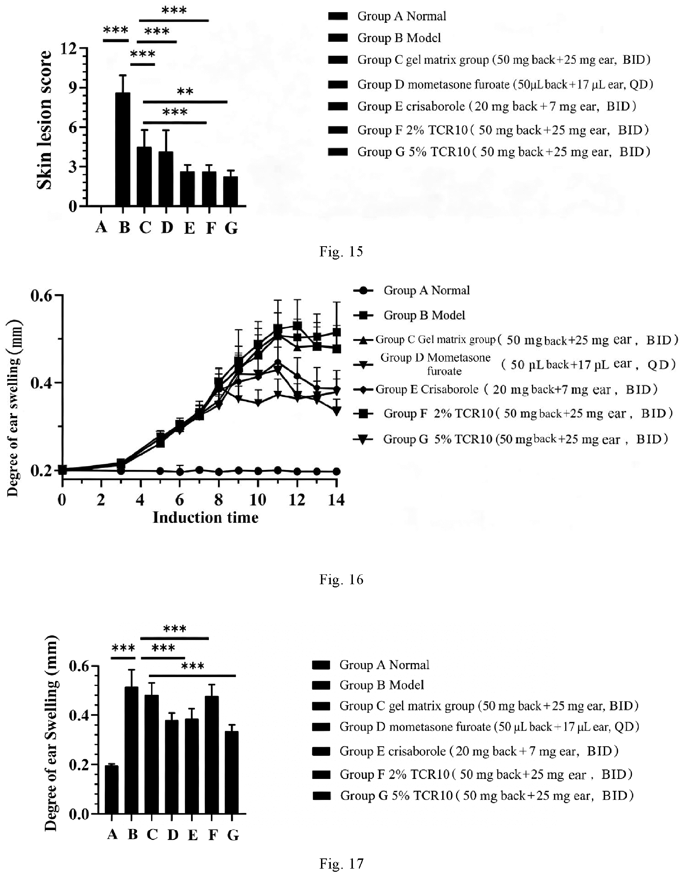

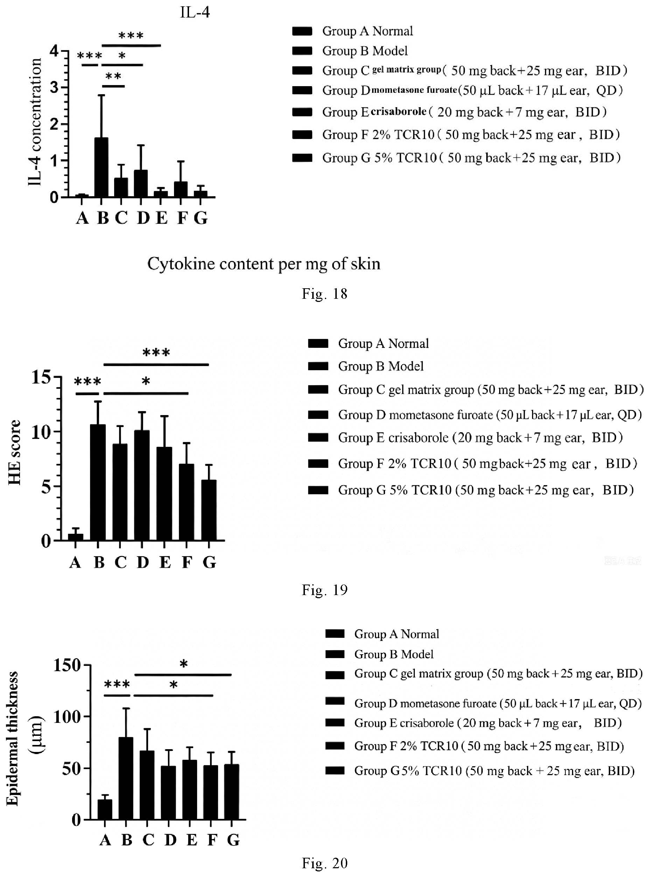

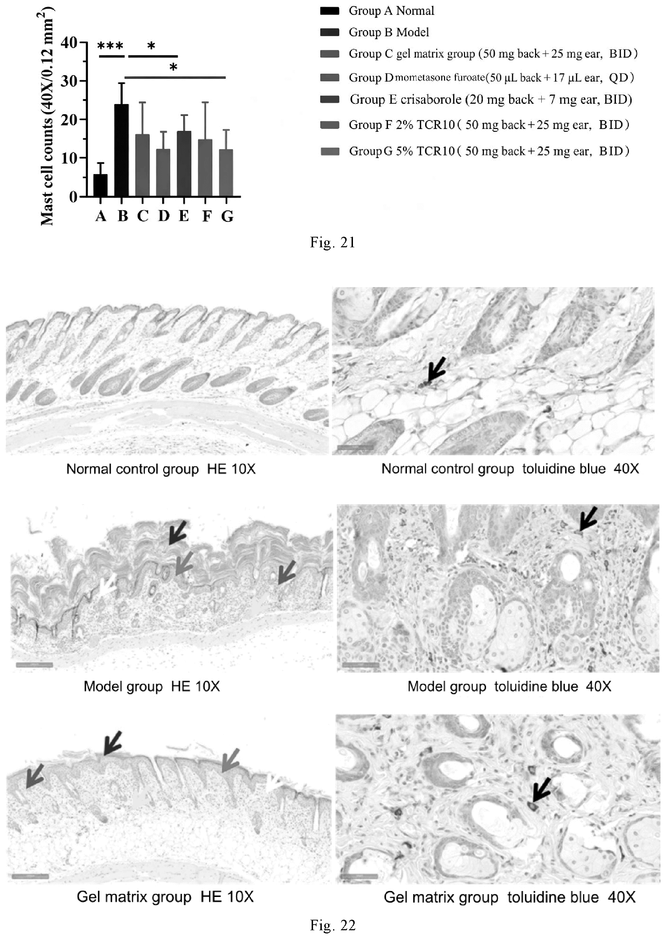

The experimental results of the gel formulation treatment model are shown in Figs. 12 to 25. Compared with the mice in the model group, the mice in the TCR10 gel group showed improvement in body weight loss (see Figs. 12 and 13), significantly reduced skin lesion scores (see Figs. 14 and 15), apparently improved ear swelling degree (see Figs. 16 and 17), apparently decreased contents of the atopic dermatitis-associated inflammatory cytokine IL-4 (see Fig. 18), significantly improved HE scores (see Fig. 19), and decreased epidermal thickness and mast cell counts (see Figs. 20 and 21).

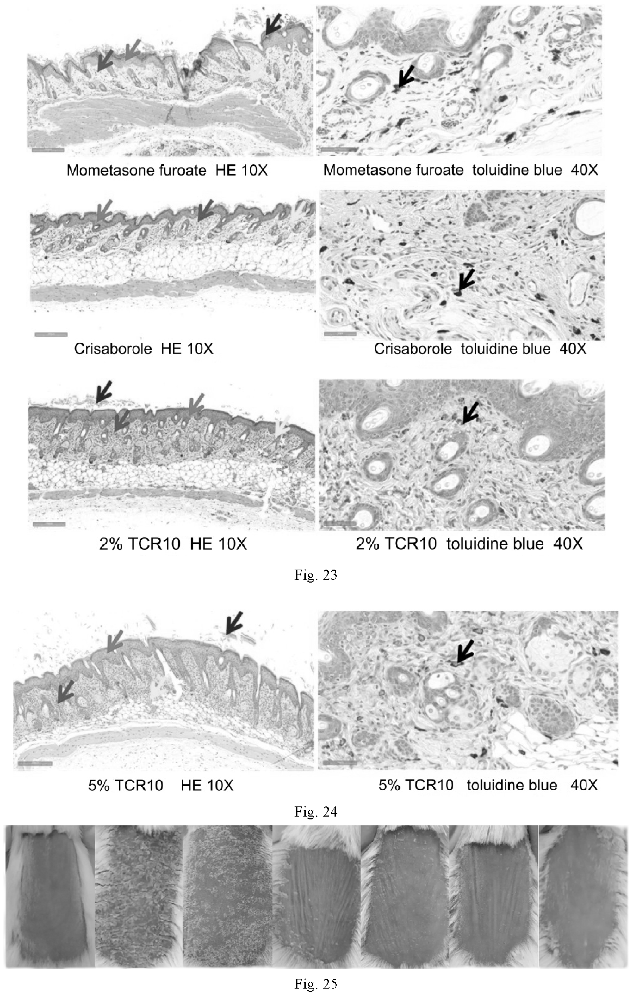

As shown in Figs. 22 to 24, the pathological condition of mice in the TCR10 gel group was significantly improved. Specifically, pathological HE staining: skins of mice in the normal control group were normal, while skins of mice in the model group showed symptoms such as obvious thickening, hyperkeratosis/parakeratosis, elongation of epidermal rete ridges and dermal congestion, inflammatory cell infiltration, and fibroplasia. Compared with the model group, skin scores of mice in the gel matrix control group (50 mg for back + 25 mg for ear, BID), mometasone furoate gel group (50 µL for back + 17 µL for ear, QD), and crisaborole ointment group (20 mg for back + 7 mg for ear, BID) did not show a significant decrease, whereas skin scores of the 2% TCR10 gel group (50 mg for back + 25 mg for ear, BID) and 5% TCR10 gel group (50 mg for back + 25 mg for ear, BID) significantly decreased, with p values of p<0.05 and p<0.001, respectively. It can be seen that the TCR10 gel formulations at tested concentrations could significantly alleviate the pathological symptoms of mice, wherein the 5% TCR10 gel group (50 mg for back + 25 mg for ear, BID) had the best therapeutic effect (see Fig. 22 to the left panel of Fig. 24).

Pathological epidermal thickness: compared with the normal control group, the epidermis of mice in the model group was significantly thickened (p<0.001). Compared with the model group, the skin epidermal thicknesses of mice in the gel matrix control group (50 mg for back + 25 mg for ear, BID) were substantially comparable, the skin epidermal thicknesses of mice in the mometasone furoate gel group (50 µL for back + 17 µL for ear, QD) and crisaborole ointment group (20 mg for back + 7 mg for ear, BID) showed a decreasing trend, but the difference was not significant; epidermal thickness of the 2% TCR10 gel group (50 mg for back + 25 mg for ear, BID) and 5% TCR10 gel group (50 mg for back + 25 mg for ear, BID) decreased significantly, with p values of p<0.05 and p<0.05, respectively, showing obvious improvement effects. Compared with the gel matrix control group, there was a decreasing trend after the treatment in the TCR10 gel group, indicating that TCR10 gel formulation can improve epidermal thickening (see Fig. 20).

As shown in Fig. 25, the crisaborole ointment group, 2% TCR10 gel group and 5% TCR10 gel group all could improve the dorsal skin lesions of mice, wherein the 5% TCR10 gel group had the best improvement effect.

As tested according to the above methods, TCR01 to TCR08, TCR11 to TCR153, and TCR155 to TCR200 all showed substantially comparable effects in improving atopic dermatitis as TCR09 and TCR10, exhibiting reduced skin lesion scores, improved body weight loss, and significantly decreased contents of atopic dermatitis-associated inflammatory cytokine (IL-4). After treatment, the keratinized layer could be observed in the dorsal epidermis, collagen fibers in the dermis were arranged neatly, and subcutaneous tissue located beneath the dermis consisting of loose connective tissue, adipose tissue and muscle layer, with no obvious abnormalities observed.

### References

1. Gombert M. et al., CCL1-CCR8 interactions: an axis mediating the recruitment of T cells and Langerhans-type dendritic cells to sites of atopic skin inflammation. J Immunol. 2005 Apr 15; 174(8):5082-91
2. Homey, B. et al., Modulation of Chemokines by Staphylococcal Superantigen in Atopic Dermatitis. Chem Immunol Allergy. 2007, 93:181-194.

For the purposes of description and disclosure, all patents, patent applications, and other publications are hereby expressly incorporated into the present application by reference. These publications are provided only because their disclosures are earlier than the filing date of the present application. All statements regarding the dates of these documents or descriptions regarding the contents of these documents are based on the information available to the applicant, and do not constitute any admission of the correctness of the dates of these documents or the contents of these documents. Moreover, in any country, any reference to these publications herein does not constitute an admission that the publication is part of the common knowledge in the art.

Those skilled in the art will recognize that the scope of the present application is not limited to the various specific embodiments and examples described above, but various modifications, substitutions, or recombinations can be made without departing from the spirit of the present application, all of which fall within the protection scope of the present application.

## Claims

1. A CCR8 antagonistic polypeptide compound, comprising an amino acid sequence selected from:
(1) H₂N-AIB-Arg-Pro-Asp-Lys-Ala-Ala-Leu-Gly-Tyr-Gln-Lys-Arg-Pro-Leu-Pro-Gln-Xaa₁-Xaa₂-Xaa₃-Xaa₄-Xaa₅-Xaa₆-CONH₂ (SEQ ID NO: 1),
wherein Xaa₁ is Val, Ala, Leu, or Ile, Xaa₂ is Leu, or Val, Xaa₃ is Leu, Val, or Ile, Xaa₄ is Thr, Ser, Ala, or Ile, Xaa₅ is Ser, Thr, or Ala, and Xaa₆ is Ile, Val, Leu, AIB, Cys, Nle, Ala, Met, or Abu;
(2) H₂N-AIB-Arg-Pro-Asp-Lys-Ala-Ala-Leu-Gly-Tyr-Gln-Lys-Arg-Pro-Ile-Pro-Asn-Ile-Xaa₇-Xaa₈-Xaa₉-Xaa₁₀-Xaa₁₁-CONH₂ (SEQ ID NO: 2),
wherein Xaa₇ is Leu, or Val, Xaa₈ is Leu, Val, or Ile, Xaa₉ is Thr, Ala, or Ser, Xaa₁₀ is Thr, Ala, or Ser, and Xaa₁₁ is Leu, Val, AIB, Cys, Nle, Ala, Met, or Abu;
(3) H₂N-AIB-Arg-Pro-Asp-Lys-Ala-Ala-Leu-Gly-Tyr-Asn-Orn-Arg-Pro-Ile-Pro-Asn-Ile-Xaa₁₂-Xaa₁₃-Xaa₁₄-Xaa₁₅-Xaa₁₆-CONH₂ (SEQ ID NO: 3),
wherein Xaa₁₂ is Leu, or Val, Xaa₁₃ is Leu, Val, or Ile, Xaa₁₄ is Thr, Ala, or Ser, Xaa₁₅ is Thr, Ala, or Ser, and Xaa₁₆ is Leu, Val, AIB, Cys, Nle, Ala, Met, or Abu;
(4) H₂N-AIB-Arg-Pro-Xaa₁₇-Xaa₁₈-Xaa₁₉-Ala-Leu-Gly-Tyr-Asn-Orn-Arg-Pro-Leu-Pro-Gln-Val-Leu-Leu-Xaa₂₀-Xaa₂₁-Abu-CONH₂ (SEQ ID NO: 4),
wherein Xaa₁₇-Xaa₁₈-Xaa₁₉ is Asp-Lys-Ala, or Glu-Orn-Ser, Xaa₂₀ is Thr, Ala, or Ser, and Xaa₂₁ is Thr, or Ser; or
(5) H₂N-AIB-Arg-Pro-Glu-Orn-Ser-Ala-Leu-Gly-Tyr-Asn-Orn-Arg-Pro-Ile-Pro-Asn-Ile-Xaa₂₂-Xaa₂₃-Xaa₂₄-Xaa₂₅-Xaa₂₆-CONH₂ (SEQ ID NO: 5),
wherein Xaa₂₂ is Leu, or Val, Xaa₂₃ is Leu, Val, or Ile, Xaa₂₄ is Thr, Ala, or Ser, Xaa₂₅ is Thr, Ala, or Ser, and Xaa₂₆ is Leu, Val, AIB, Cys, Nle, Ala, Met, or Abu;
or an amino acid sequence having at least 80% identity thereto.

2. The polypeptide compound of claim 1, wherein Xaa₁-Xaa₂-Xaa₃ is Val-Leu-Leu; and/or
Xaa₄ is Thr, Ser, or Ala; and/or
Xaa₅ is Ser, Thr, or Ala; and/or
Xaa₆ is Ile, Val, Leu, AIB, Cys, Nle, Ala, Met, or Abu.

3. The polypeptide compound of claim 1 or 2, wherein Xaa₁-Xaa₂-Xaa₃ is Val-Leu-Leu, Xaa₄ is Thr, or Ser, Xaa₅ is Ser, and Xaa₆ is Ile, Val, AIB, Cys, Nle, Ala, Met, or Abu; or
Xaa₁-Xaa₂-Xaa₃ is Val-Leu-Leu, Xaa₄ is Thr, or Ser, Xaa₅ is Ser, Thr, or Ala, and Xaa₆ is Ile.

4. The polypeptide compound of claim 1 or 2, wherein Xaa₁-Xaa₂-Xaa₃-Xaa₄ is Val-Leu-Leu-Thr, Xaa₅ is Ser, Thr, or Ala, and Xaa₆ is Val, Leu, AIB, Cys, Nle, Ala, Met, or Abu; or
Xaa₁-Xaa₂-Xaa₃-Xaa₄ is Val-Leu-Leu-Ser, Xaa₅ is Ser, or Thr, and Xaa₆ is Ile, Val, AIB, Cys, Nle, Ala, Met, or Abu.

5. The polypeptide compound of any one of claims 1-4, wherein the polypeptide compound comprises the following amino acid sequence:
H₂N-AIB-Arg-Pro-Asp-Lys-Ala-Ala-Leu-Gly-Tyr-Gln-Lys-Arg-Pro-Leu-Pro-Gln-Val-Leu-Leu-Xaa₂₇-Ser-Val-CONH₂ (SEQ ID NO: 6), wherein Xaa₂₇ is Thr, or Ser; or
H₂N-AIB-Arg-Pro-Asp-Lys-Ala-Ala-Leu-Gly-Tyr-Gln-Lys-Arg-Pro-Leu-Pro-Gln-Val-Leu-Leu-Ser-Ser-Xaa₂₈-CONH₂ (SEQ ID NO: 206), wherein Xaa₂₈ is Ile, or Val.

6. The polypeptide compound of claim 1, wherein the polypeptide compound comprises the amino acid sequence set forth in any one selected from SEQ ID NO: 7 to SEQ ID NO: 205; preferably, the amino acid sequence of the polypeptide compound is set forth in SEQ ID NO: 16, or SEQ ID NO: 15.

7. The polypeptide compound of claim 1, wherein
(1) Xaa₇-Xaa₈ is Leu-Leu; preferably, Xaa₉ is Thr, or Ser, Xaa₁₀ is Ser, or Thr, and Xaa₁₁ is Val;
(2) Xaa₁₂-Xaa₁₃ is Leu-Leu; preferably, Xaa₁₄ is Thr, or Ser, Xaa₁₅ is Ser, or Thr, and Xaa₁₆ is Val;
(3) Xaa₁₇-Xaa₁₈-Xaa₁₉ is Asp-Lys-Ala, Xaa₂₀ is Thr, or Ser, and Xaa₂₁ is Ser, or Thr; or
(4) Xaa₂₂-Xaa₂₃ is Leu-Leu; preferably, Xaa₂₄ is Thr, or Ser, Xaa₂₅ is Ser, or Thr, and Xaa₂₆ is Val.

8. A pharmaceutical composition, comprising the polypeptide compound of any one of claims 1-7 and a pharmaceutically acceptable excipient.

9. The pharmaceutical composition of claim 8, wherein the pharmaceutically acceptable excipient comprises solvents, propellants, solubilizers, cosolvents, emulsifiers, colorants, disintegrants, fillers, lubricants, wetting agents, osmotic pressure regulators, stabilizers, glidants, flavoring agents, preservatives, suspending agents, antioxidants, penetration enhancers, pH adjusters, surfactants, or diluents;
preferably, the pharmaceutical composition is a powder, a granule, a film-coating agent, a cream, an ointment, a gel, a lotion, a foam, a patch, a liniment, an elixir, an aerosol, an emulsion, a solution, or a suspension.

10. The pharmaceutical composition of claim 8 or 9, wherein the pharmaceutical composition further comprises an additional therapeutic agent for atopic dermatitis, wherein the additional therapeutic agent for atopic dermatitis is selected from glucocorticoids, calcineurin inhibitors, PDE4 inhibitors, JAK inhibitors, antibiotics, anti-pruritic drugs, wet compress drugs, calamine lotion or zinc oxide cream, antihistamines, antiviral drugs, and immunosuppressants.

11. A kit, comprising the polypeptide compound of any one of claims 1-7, or the pharmaceutical composition of any one of claims 8-10.

12. A polypeptide compound of any one of claims 1-7, a pharmaceutical composition of any one of claims 8-10, or a kit of claim 11 for use in preventing or treating cancers or tumors, inflammatory diseases, viral infections, IgG4-related diseases, cutaneous immune diseases, or related symptoms thereof.

13. The polypeptide compound, pharmaceutical composition, or kit for use of claim 12, wherein the tumor is selected from solid tumors, more preferably multiple myeloma, lymphoma, or melanoma; the cancer is selected from esophageal cancer, lung cancer, non-small cell lung cancer, gastric cancer, head and neck squamous cell carcinoma, liver cancer, breast cancer, cervical cancer, ovarian cancer, colorectal cancer, colon cancer, rectal cancer, kidney cancer, bladder cancer, dermatofibrosarcoma, osteosarcoma, pancreatic cancer, and prostate cancer, the inflammatory disease is selected from asthma and allergic enteritis, the IgG4-related disease is selected from sialadenitis, the cutaneous immune disease or related symptoms thereof are cutaneous immune diseases related to the CCL1-CCR8 signaling pathway or related symptoms thereof; more preferably, the cutaneous immune disease or related symptoms thereof are selected from eczema, atopic dermatitis, psoriasis, lupus erythematosus, scleroderma, dermatomyositis, pemphigus, bullous pemphigoid, dermatitis herpetiformis, epidermolysis bullosa, and related symptoms thereof.

14. The polypeptide compound, pharmaceutical composition, or kit for use of claim 12 or 13, wherein the cutaneous immune disease or related symptoms thereof are selected from atopic dermatitis, and related symptoms thereof; even more preferably, the atopic dermatitis is infantile atopic dermatitis, childhood atopic dermatitis, or adolescent and adult atopic dermatitis, or the atopic dermatitis is mild, moderate, or severe atopic dermatitis.

15. A polypeptide compound of any one of claims 1-7, a pharmaceutical composition of any one of claims 8-10 or a kit of claim 11 for use in blocking the CCL1-CCR8 signaling pathway.
